# EUROPEAN PATENT SPECIFICATION

(11) **EP 3 137 038 B1**
(45) Date of publication and mention of the grant of the patent: **30.12.2020**
(21) Application number: 15785983.6
(22) Date of filing: 28.04.2015
(51) Int. Cl.: G16H 10/00, A61J 1/03, A61J 7/04, A61F 9/00

(54) **UNOBTRUSIVE WIRELESS ELECTRONIC SYSTEMS FOR MONITORING AND FACILITATING PATIENT COMPLIANCE**
UNAUFFÄLLIGE DRAHTLOSE ELEKTRONISCHE SYSTEME ZUR ÜBERWACHUNG UND ERMÖGLICHUNG DER PATIENTENCOMPLIANCE
SYSTÈMES ÉLECTRONIQUES SANS FIL DISCRETS DESTINÉS À SURVEILLER ET À FACILITER L'OBSERVANCE DU PATIENT

(30) Priority: 28.04.2014 US 201461985384 P
(43) Date of publication of application: 08.03.2017
(73) Proprietor: The Regents of the University of California, Oakland, CA 94607-5200 (US)
(72) Inventor: COLEMAN, Todd, Prentice, La Jolla, CA 92037 (US); WEINREB, Robert, N., Rancho Santa Fe, CA 92066 (US); KIM, Yun, Soung, San Diego, CA 92122 (US); BAJEMA, Michael, San Diego, CA 92122 (US); KANG, Dae, Los Angeles, CA 90007 (US); OMAR, Amr, Haj, San Diego, CA 92122 (US)
(74) Representative: Potter Clarkson
(86) International application number: PCT/US2015/028075
(87) International publication number: WO 2015/168171

(56) References cited:
- WO-A1-2013/127564
- WO-A1-2013/127564
- WO-A2-2007/077224
- WO-A2-2007/077224
- US-A- 5 412 372
- US-A1- 2002 067 270
- US-A1- 2002 067 270
- US-A1- 2005 162 979
- US-A1- 2006 231 109
- US-A1- 2006 231 109
- US-A1- 2008 114 490
- US-A1- 2008 114 490
- US-A1- 2011 050 431
- US-A1- 2011 050 431
- Wikipeddia: "Comparator", Wikipedia, 19 April 2014 (2014-04-19), XP055408651, Retrieved from the Internet: URL:https://en.wikipedia.org/w/index.php?t itle=Comparator&oldid=604831956 [retrieved on 2017-09-21]

## Description

### TECHNICAL FIELD

This patent document relates to systems, devices, and processes that use thin flexible electronic sensor technologies.

### BACKGROUND

Flexible electronics can include electric circuits and devices formed on flexible substrates that can be applied to and conform to a variety of surface geometries. For example, flexible electronics have a key advantage that they can wrap arbitrary, curvilinear surfaces and, at the same time, achieve mechanical properties that approach those of the carrier substrate that the flexible electronics are mounted or integrated. WO 2007/077224 describes dispensers of unit dose medications which gather and process information on patient compliance with a desired dispensing regimen. A detector generates a signal each time a unit dose is dispensed. A flexible substrate physically supports and connects the detector and a processor into a unitary structure and conducts the signal from the detector to the processor. US 5412372 (A) provides a portable medication dispenser for monitoring use of medications provided in standard as-marketed blister packages. A standard blister package and a separate, disposable sensor sheet are simply and easily loaded into a compact housing containing supervisory electronics, visual indicators, and an audible alarm. The sensor sheet consists of electrical or optical sensing regions corresponding to each individual compartment of the blister package.

### SUMMARY

The scope of the invention is determined by the appended independent claims. Preferable embodiments are determined by the appended dependent claims. Aspects, implementations, and examples that do not fall within the scope of the claims are to be interpreted as useful for understanding the invention. Techniques, systems, and devices are disclosed for implementing unobtrusive wireless electronics on containers of medical substances (e.g., prescription drugs) for monitoring and facilitating patient compliance. In some implementations, for example, flexible electronic sensor and wireless communication systems are employed on pharmaceutical bottles or containers, e.g., including patient alert features, for assisting patient compliance of medication schedules.

In one aspect of the disclosure, a device for monitoring patient compliance of a medication includes a substrate that is attachable and conformable to a container containing medicine; a data acquisition unit including a sensor and a signal processing circuit formed on the substrate, in which the sensor is operable to transduce an occurrence of the medicine dispensing from the container into an electrical signal, and the signal processing circuit is operable to amplify the electrical signal; a data processing unit including a processor and a memory formed on the substrate and in communication with the data acquisition unit to process the amplified electrical signal as data; and a communications unit formed on the substrate and in communication with the data processing unit to wirelessly transmit the processed data to a remote device.

In one aspect of the disclosure, a method for monitoring dispensing of medicine from a container includes detecting a signal by a sensor on a conformal substrate attached and conformed to a container containing medicine; and processing the detected signal to produce data indicative of an occurrence or a non-occurrence of the medicine dispensed from the container.

In one aspect of the disclosure, a device for monitoring dispensing of a fluid from a container includes a data acquisition unit including a sensor and a signal processing circuit formed in a material structure of a container capable of storing and dispensing a fluid, in which the sensor transduces an occurrence of the fluid dispensing from the container into an electrical signal, and the signal processing circuit amplifies the electrical signal; a data processing unit including a processor and a memory unit, the data processing unit formed in the material structure of the container and in communication with the data acquisition unit to process the amplified electrical signal as data to determine a dispensing event of the fluid from the container; and a communications unit formed in the material structure of the container and in communication with the data processing unit to wirelessly transmit the processed data to a remote device.

In one aspect of the disclosure, a device for monitoring dispensing from a container includes a substrate that is attachable and conformable to a container; a data acquisition unit including a sensor and a signal processing circuit formed on the substrate, in which the sensor is operable to transduce an occurrence of a content contained in the container dispensing from the container into an electrical signal, and the signal processing circuit is operable to amplify the electrical signal; and a communications unit formed on the substrate and in communication with the signal processing circuit to wirelessly transmit the amplified electrical signal to a remote device.

Those and other features are described in greater detail in the drawings, the description and the claims.

### BRIEF DESCRIPTION OF THE DRAWINGS

FIG. 1A shows a block diagram of an exemplary flexible electronics system of the disclosed technology to monitor dispensing of a fluid from a container by a user.
FIG. 1B shows a block diagram of an exemplary data processing unit of the exemplary flexible electronics system.
FIG. 1C shows an illustrative diagram of the exemplary flexible electronics system shown in FIG. 1A.
FIGS. 1D-1G show block diagrams of an exemplary method to monitor dispensing of medicine from a container.
FIGS. 2A and 2B show diagrams of an exemplary temperature data acquisition unit of a flexible electronics system of the disclosed technology unobtrusively attached to a droplet dispenser to monitor droplet dispensing from a container.
FIGS. 2C-2F show images and diagrams of exemplary flexible temperature sensor systems to monitor dispensing of a droplet from a container nozzle.
FIGS. 3A and 3B show diagrams of an exemplary optical data acquisition unit of a flexible electronics system of the disclosed technology unobtrusively attached to a droplet dispenser to monitor droplet dispensing from a container.
FIGS. 3C-3D show diagrams and images of exemplary flexible optical sensor systems to monitor dispensing of a droplet from a container nozzle.
FIGS. 4A and 4B show diagrams and images of an exemplary system to monitor dispensing of a droplet using an exemplary flexible sensor unit including a force/pressure sensor attached to a droplet dispenser.
FIG. 4C shows an image of an exemplary force and/or pressure data acquisition unit of the disclosed technology.
FIG. 4D shows a diagram of the exemplary force/pressure data acquisition unit integrated with a label wrapped around a squeezable container.
FIGS. 4E and 4F show diagrams of an exemplary flexible signal processing circuit to process detected force or pressure.
FIG. 4G shows a sequence of images depicting an implementation of the exemplary force/pressure data acquisition unit including an optional LED to indicate that force or pressure is being applied.
FIG. 4H shows an illustrative diagram of an exemplary pressure and position data acquisition unit of the disclosed technology unobtrusively attached to a container having a detachable lid to detect the dispensing of the contents from the container.
FIGS. 5A-5C show diagrams of an example user interface of a software application of the disclosed technology.
FIG. 6 shows a flow diagram of an exemplary feedback method to facilitate patient compliance in taking a medication using a bottle incorporating an exemplary flexible electronic monitoring system of the disclosed technology.
FIG. 7 shows an illustrative diagram of a method to produce a blister pack container including an exemplary flexible electronics system of the disclosed technology.
FIG. 8 shows an illustrative diagram of a patient using a smart blister pack medicine container of the disclosed technology.

### DETAILED DESCRIPTION

Patient compliance for use of medications or other controlled substances is an important part of a medical treatment or healthcare of a patient. Currently, the ability to monitor patient compliance is limited by several factors. For example, most bottles or containers of medications lack a built-in monitoring system, and thus physicians and other caregivers cannot determine how the patient is doing. Also, the relatively few bottles or containers that include some monitoring mechanisms tend to have rigid, thick, and/or heavy components (e.g., such as electronics) that render the bottle/container in an obtrusive form factor and thus compromise the convenience for the patient in normal situations. If the ergonomics of the bottle are too unobtrusive or too heavy (e.g., where the bottle cannot fit in the user's pocket, purse or other carrier or not even convenient to carry in hand), then such compliance systems may not receive acceptance by patients and thus defeat the point of their own use - or worse, such obtrusive or cumbersome compliance monitoring systems may cause non-compliance. There is a significant unmet need for accurate patient compliance in a manner that has the same form factor or a very close form factor to the bottle/container/pack of medicine that otherwise does not include compliance monitoring technologies. Moreover, in the case of drop dispensing bottles, millions of dollars have already been spent on use-cases and studies to determine the optimal bottle size, shape, etc. for not only delivering accurate drops, but for maximizing patient utility. Therefore, it is important that patient compliance monitoring systems are unobtrusively integrable into such optimized container designs.

The disclosed technology describes multiple approaches and system designs using ultra-thin flexible electronics to inconspicuously monitor patient compliance, and provide avenues to intervene and encourage such compliance. The disclosed ultra-thin flexible electronics techniques, systems, and devices can be employed in existing designs of medicine bottles, blister packs and other type containers, which obviate the need to study, develop and optimize new types of bottles (i.e., start from the drawing board in terms of patient utility).

In some examples, the disclosed technology uses flexible electronics and wireless communications telemetry to monitor when a drop is dispensed from a bottle or a pill is dislodged from a blister pack, stores the data associated with the patient's monitored action in memory (e.g., such as a local memory in the flexible electronics), and transmits to a data processing device, e.g., such as a user's mobile communications device (e.g., smartphone, tablet, or wearable computing/communications device like a 'smartwatch', 'smartglasses', etc.). Moreover, in some examples, the disclosed flexible electronics unit unobtrusively employed to the medicine container may include LED indicators or other indicators or transducers and logic inside the flexible electronics circuits to adapt to when events have occurred and provide sensory cues (e.g., visual, auditory, or haptic reminders) to the subject.

Techniques, systems, and devices are disclosed for implementing unobtrusive wireless electronics on containers of medical substances (e.g., including prescription drugs) for monitoring and facilitating patient compliance.

In one aspect, a device for monitoring the dispensing of a medicine from a medicine container includes a data acquisition unit, and a wireless communications unit formed on a conformal substrate that is attached to a container containing medicine such that the device is inconspicuously integrated with the container. In some examples, the device includes a data processing unit formed on the conformal substrate and in communication with the data acquisition and the wireless communication unit. In some implementations, the substrate includes a mechanically flexible (e.g., stretchable and bendable) and an electrically insulative material. The data acquisition unit includes a sensor and a signal processing circuit, in which the sensor is operable to transduce an occurrence of the medicine dispensing from the container into an electrical signal, and the signal processing circuit is operable to amplify the electrical signal. The communications unit is in communication with the signal processing circuit and/or the data processing unit to wirelessly transmit the amplified signal or the processed data to a remote device. The data processing unit is in communication with the data acquisition unit and includes a processor and a memory to process the amplified electrical signal as data.

In some implementations of the disclosed flexible electronic sensor and wireless communication technology, the device can be applied to or integrated into bottles or other type containers of various types of fluids, e.g., including, but not limited to, prescription and nonprescription drugs such as eye drops. The disclosed flexible electronic device can be used to monitor the usage of such fluids by a user of the bottle or container. For example, in some implementations, the flexible electronic device can include user alert communication modules that provide communicative signals from the device to the user, e.g., for assisting patient compliance of medication schedules. For example, the flexible electronic device can be used to infer the release of medication from an eye drop bottle to which the device is applied or integrated. The dispensing time can be recorded by sensors and processed by the device, which can be used to provide the user with real-time feedback to encourage patient compliance. Also, for example, the disclosed flexible electronic sensor device can be utilized to assess adherence and compliance in the evaluation of new drugs during clinical research trials.

Current state of the art sensor systems are obtrusive to a user by making the bottle bulkier. Also, many existing sensor systems are specific to a certain medicine, i.e., interact with the medication. Conversely, the disclosed flexible electronic sensor system is designed to be implemented within any conformation or geometry of existing bottles, e.g., which in some implementations can be wrapped around the bottle with minimal obtrusion using thin, flexible electronics. For example, the flexible electronic units of the disclosed technology can be incorporated with existing medicine container droplet bottles, blister packs, or other container forms such that flexible electronic units retain the look, feel, and operation of the medicine container. Implementation of the disclosed technology does not change or create complications in the medicine container design, medicine container fabrication process, or the medicine's regulatory process. For example, the disclosed flexible electronics units can be implemented on medicine containers such that the flexible electronics units do not interact with the medication.

In one example, an exemplary flexible electronics patient compliance device can be integrated with a labeling wrapped around a droplet bottle for a particular medicine (e.g., eye drops), or attached and conformed on the droplet bottle after the medicine is enclosed prior to or concurrently with application of the labeling, where the labeling can be attached over or away from the exemplary flexible electronics patient compliance device. The exemplary device can be applied to the exterior of the medicine container after the medicine has been enclosed or sealed within the container.

### Examples

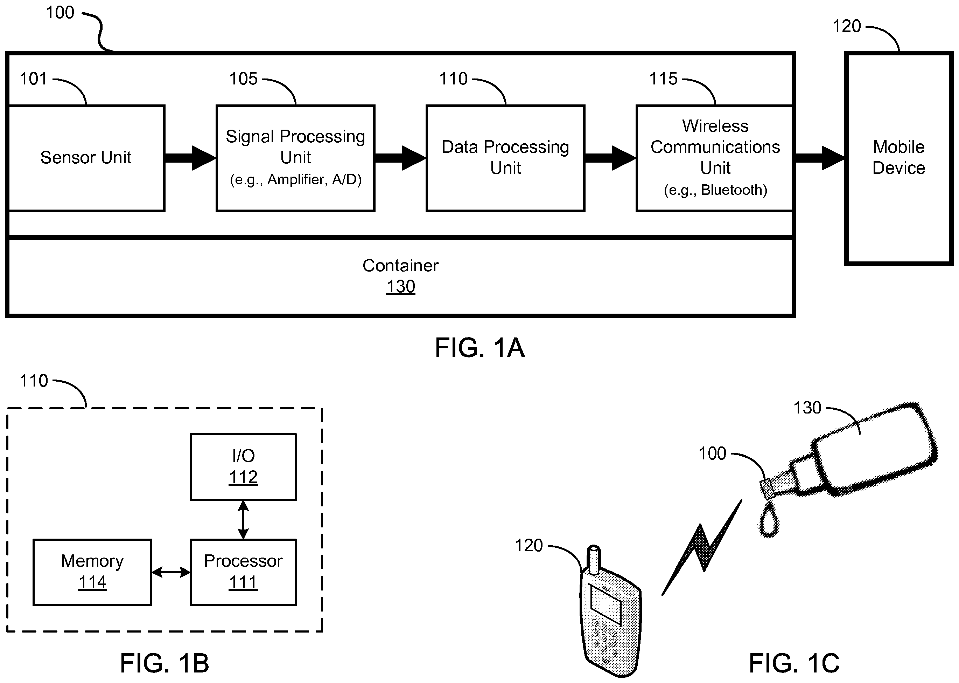

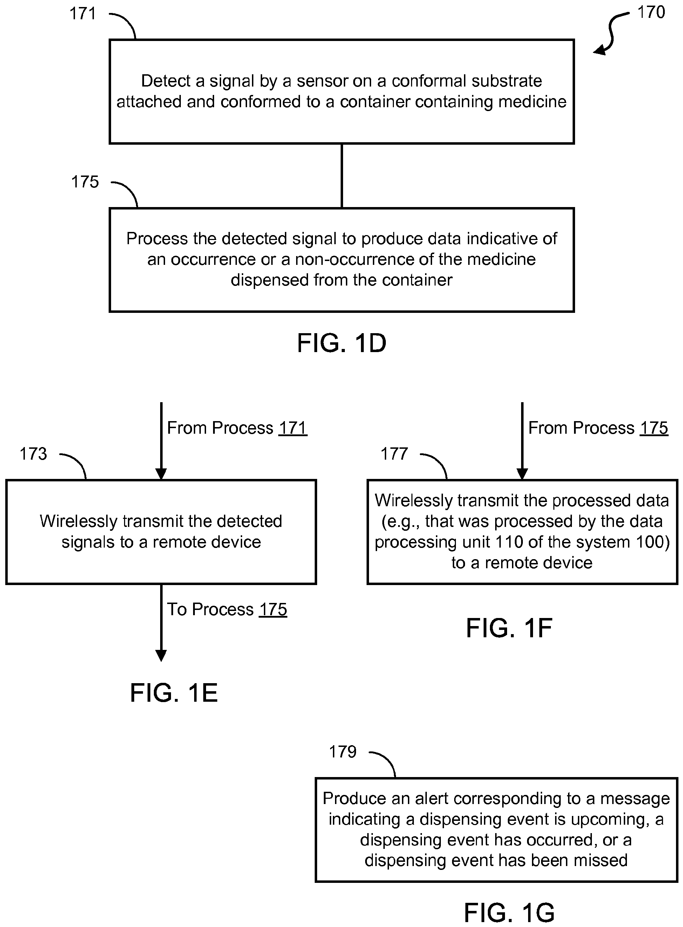

FIG. 1A shows a diagram of an exemplary flexible electronics system 100 to monitor dispensing of a fluid from a container 130 by a user (e.g., a patient). The system 100 includes a sensor unit 101 to transduce an occurrence of use of the container 130 into an electrical signal, e.g., including the dispensing of an amount of fluid and/or change in orientation of the container 130. The system 100 includes a signal processing unit 105 to process the captured electrical signals of the sensor unit 101, e.g., such as amplification and other signal conditioning, and/or conversion from an analog signal to a digital signal. The system 100 includes a data processing unit 110 including a processor and a memory to process and store or buffer the processed signals as data. The system 100 includes a wireless communication unit 115 to transmit the processed data to another remote device, e.g., such as a mobile communications device 120, as shown in FIG. 1A. For example, the wireless communications unit 115 can be configured to transmit and receive data from the remote device. FIG. 1C shows an illustrative diagram of the exemplary flexible electronics system 100 inconspicuously attached to the container 130 and in wireless communication with the mobile communications device 120.

The system 100 can be configured in wireless communications with the mobile communications device 120 of the user, e.g., to provide an alert to the user to utilize the fluid in the container 130, e.g., such as a medication. For example, the system 100 can be used to record information about the timing and/or quantity of fluid dispensed (e.g., such a drop), in which this information is logged with circuitry attached on the container 130 and used to provide the user for feedback on his/her use, e.g., via the mobile communications device 120.

In some implementations, for example, the flexible electronics system 100 can be configured in a device package including a substrate that includes a mechanically flexible material (e.g., bendable and/or stretchable) structured to mechanically conform to and/or adhere to the exterior of the container 130 to substantially match the form factor of the container 130, or to mechanically conform to and/or integrate within the material of the container 130. For example, the substrate can include, but is not limited to, polydimethylsiloxane (PDMS), consumer grade adhesives (e.g., 3M Scotch® Tape and Tegaderm™), and other thin film materials including silicon-based, polyimide-based thin films. The substrate can be configured to have a thickness in a range of a few millimeters to tens of microns, e.g., such as 10 µm thickness. For example, the device package of the system 100 can be configured to have a thickness approximate to that of a piece of paper (e.g., 50 µm). The system 100 can be attached to the container 130 in a variety of examples. The sensor unit 101, the signal processing unit 105, the data processing unit 110, and the wireless communications unit 115, are implemented in a single device package attached to the container 130.

In some implementations, for example, the signal processing unit 105 can include transistors, capacitors, resistors, inductors, transistors, diodes, amplifiers, and/or other circuit elements, etc., to process the captured signals acquired by the sensor unit 101. Some examples of the signal processing unit 105 are described later.

FIG. 1B shows a block diagram of an exemplary data processing unit of the exemplary flexible electronics system. As shown in FIG. 1B, the data processing unit 110 can include a processor 111 to process data and a memory 114 in communication with the processor 111 to store data. For example, the processor 111 can include a central processing unit (CPU) or a microcontroller unit (MCU). For example, the memory 114 can include processor-executable code, which when executed by the processor 111, configures the data processing unit 110 to perform various operations, such as receiving information, commands, and/or data, processing information and data, and transmitting or providing information/data to another entity or to a user. For example, the data processing unit can include a power supply to provide power to components of the system 100, e.g., such as unobtrusive batteries, autonomously generated power supply units (e.g., such as photovoltaic cells) or wireless powering units.

To support various functions of the data processing unit 110, the memory 114 can store other information and data, such as instructions, software, values, images, and other data processed or referenced by the processor 111. Various types of Random Access Memory (RAM) devices, Read Only Memory (ROM) devices, Flash Memory devices, and other suitable storage media can be used to implement storage functions of the memory 114. The memory 114 can store data and information of the data processing unit 110 and other units of the system 100, e.g., including the communications unit 115, the sensor unit 101, and/or the signal processing unit 105, as well as information about other systems and devices in communication with the system 100. For example, the memory 114 can store device unit parameters, and hardware constraints, as well as software parameters and programs of the mobile device 120.

The data processing unit 110 can include an I/O unit 112 that can allow communicative connectability of the data processing unit 110 to other units of the system 100. For example, the data processing unit 110 can be configured in communications with other units of the system 100, e.g., which can be configured in a separate casing or module attached to container 130, using various types of wired or wireless interfaces compatible with typical data communication standards, for example, including, but not limited to, Universal Serial Bus (USB), IEEE 1394 (FireWire), Bluetooth, IEEE 802.111, Wireless Local Area Network (WLAN), Wireless Personal Area Network (WPAN), Wireless Wide Area Network (WWAN), WiMAX, IEEE 802.16 (Worldwide Interoperability for Microwave Access (WiMAX)), 3G/4G/LTE cellular communication methods, and parallel interfaces. The I/O unit 112 can also provide communicative connectability of the data processing unit 110 to an external interface, source of data storage, or display device. The I/O unit 112 of the data processing unit 110 can also interface with other external interfaces, sources of data storage, and/or visual or audio display devices, etc. to retrieve and transfer data and information that can be processed by the processor 111, stored in the memory 114, or exhibited on an output unit of the mobile communications device 120.

Referring back to FIG. 1A, the mobile communications device 120 can include a smartphone, tablet, or wearable communications device (e.g., such as a smartwatch, smartglasses, etc.). In some implementations, for example, the mobile communications device 120 can also include a communications receiver on a personal computer, e.g., such as a desktop or laptop computer, or on another computer system. For example, data sent to the mobile communications device 120 can be subsequently sent by the device 120 to a computer system or communication network accessible via the Internet (referred to as 'the cloud') that includes one or more remote computational processing devices (e.g., servers in the cloud). For example, various types of wired or wireless interfaces compatible with typical data communication standards can be used in communications of the system 100 with the mobile communications device 120, e.g., including, but not limited to, a cable link such as the Universal Serial Bus (USB) or IEEE 1394 (FireWire), or a wireless link based on an RF communication protocol such as a near-field communication protocol, Bluetooth, IEEE 802.111, or other communication links including, e.g., Wireless Local Area Network (WLAN), Wireless Personal Area Network (WPAN), Wireless Wide Area Network (WWAN), WiMAX, IEEE 802.16 (Worldwide Interoperability for Microwave Access (WiMAX)), 3G/4G/LTE cellular communication methods, and parallel interfaces.

The sensor unit 101 can be configured to include one or more of the following exemplary sensors on the flexible, stretchable substrate, e.g., including, but not limited to, 1) temperature sensor; 2) optical sensor; 3) force/pressure sensor; and 4) position sensor. One or multiple sensor units 101 may be unobtrusively attached to the container 130, e.g., such as one or a plurality of a particular type sensor attached to one region of the container 130, and one or a plurality of a different or the same type sensor attached to another region of the container 130. For example, a droplet dispenser container may have an optical sensor of the disclosed technology attached to the nozzle region of the dispenser and may have a pressure sensor of the disclosed technology attached to the body region (e.g., squeezable region) of the container. Also, for example, the droplet dispenser container may have a position sensor of the disclosed technology attached to the body, and/or nozzle region of the dispenser, and/or may have a temperature sensor of the disclosed technology attached to the nozzle region.

FIG. 1D shows a block diagram of a method 170 to monitor dispensing of medicine from a container, e.g., which can include monitoring patient compliance for use of the medicine. The method 170 includes a process 171 to detect a signal by a sensor on a conformal substrate attached and conformed to a container containing medicine. In some implementations of method 170, for example, the process 171 can be implemented by the system 100 unobtrusively attached to or integrated with the container containing medicine, e.g., in which the system 100 can use one or more of sensors and types of sensors on the flexible, stretchable substrate, e.g., the temperature sensor, optical sensor, force/pressure sensor, and position sensor. The method 170 includes a process 175 to process the detected signal to produce data indicative of an occurrence or a non-occurrence of the medicine dispensed from the container.

Implementations of the method 170 can include one or more of the following features. In implementations, for example, the process 171 can include transducing events corresponding to the medicine dispensing and not dispensing from the container into distinguishable electrical signals, and amplifying the electrical signals. In some implementations, for example, the process 175 can include determining an amount of the medicine dispensed from the container based on the detected signal. In some implementations, for example, the process 175 can include associating a time value with a determined occurrence, and can also include determining if the time value associated with the determined occurrence is before or after a predetermined time (e.g., such as based on a medication use schedule, which may be provided by a caregiver or based on the prescription), which can be used to indicate the degree or level of patient compliance.

In some implementations of the method 170, for example, the process 175 can be performed by the data processing unit 110 on the conformal substrate 102 of the system 100; and/or in some implementations of the method 170, for example, the process 175 can be performed by a processing unit on a remote device (e.g., such as the user's mobile device 120, and/or another remote computing device such as a server in the cloud). As shown in FIG. IE, in some implementations, the method 170 can include a process 173 to wirelessly transmitting the detected signals to the remote device that includes a processing unit to implement the process 175. As shown in FIG. IF, in some implementations, the method 170 can include a process 177 to wirelessly transmit the processed data that was processed by the data processing unit 110 of the system 100 to the remote device. As shown in FIG. 1G, the method 170 can include a process 179 to produce an alert (e.g., which can be displayed by an alert unit of the system 100 (e.g., LED or other light emitter, audio speaker, or haptic unit such as a vibrator including a motor coupled to a gear), or which can be displayed by the user's mobile device 120) corresponding to a message indicating a dispensing event is upcoming, a dispensing event has occurred, or a dispensing event has been missed.

### Temperature Sensing

In some examples, the sensor unit 101 can include one or more temperature sensors 101a (e.g., such as a temperature dependent resistive flow sensor). For example, the exemplary temperature dependent resistive flow sensors 101a can be formed using a material with temperature sensitive resistance, e.g., such as platinum. In implementations of the system 100, a temperature fluctuation occurs when liquid passes over the nozzle of the container 130. This translates to a change in resistance on the exemplary temperature dependent resistive flow sensors 101a of the system 100, which can acquire the signal and process the signal into data to indicate that an amount of the liquid (e.g., such as a drop) was released from the container 130.

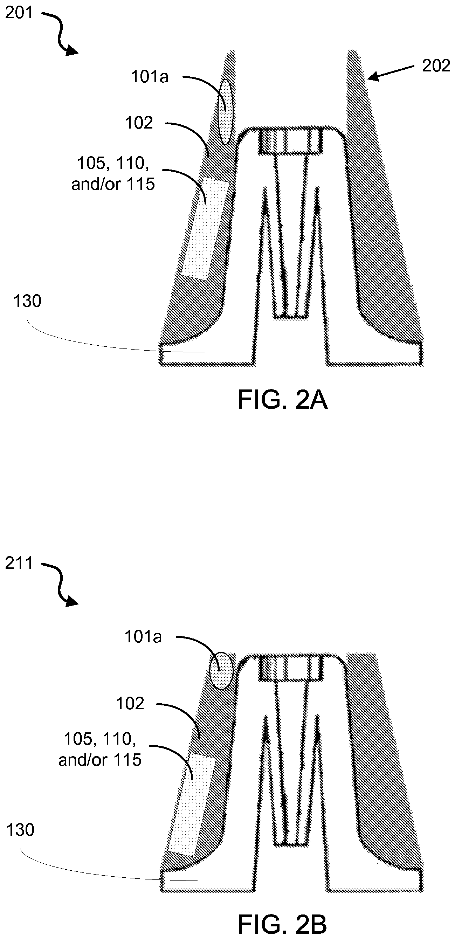

FIG. 2A shows a cross-sectional diagram of an exemplary temperature data acquisition unit 201 of the disclosed technology unobtrusively attached to a droplet dispenser to detect the dispensing of a droplet from a container, e.g., in which the detected dispensing data can be processed for monitoring patient compliance. As shown in the diagram of FIG. 2A, the temperature data acquisition unit 201 is attached to or integrated in a conformal substrate 102 of the system 100 that is attachable to the container 130, e.g., shown attached to the nozzle region of the container 130, and such that the system 100 is inconspicuously integrated with the container 130. In the example shown in FIG. 2A, a region 202 of the conformal substrate 102 can be configured to partially protrude past the opening of the nozzle of the container 130. The temperature data acquisition unit 201 includes a temperature sensor 101a, e.g., such as a temperature-dependent resistive flow sensor, configured on the conformal substrate 102 in the region 202. In some implementations, for example, the temperature sensor 101a can be embedded on an interior side of the substrate 202 of the region 202 such that a dispensed droplet passes through the interior passage of the region 202 to cause a temperature-based change on the temperature sensor 101a, e.g., such as change in resistance on the exemplary temperature-dependent resistive flow sensor. The temperature sensor 101a is in communication with the signal processing circuit 105, e.g., which can amplify and/or modulate the detected signal for data processing by the data acquisition unit 110, and/or for transmission to the device 120 by the wireless communication unit 115.

FIG. 2B shows a cross-sectional diagram of another exemplary temperature data acquisition unit 211 of the disclosed technology unobtrusively attached to a droplet dispenser to detect the dispensing of a droplet from a container. As shown in the diagram of FIG. 2B, the temperature data acquisition unit 211 is attached to or integrated in the conformal substrate 102 of the system 100 that is attachable to the nozzle of the container 130, such that the temperature sensor 101a is configured on the conformal substrate 102 proximate the tip of the nozzle to detect a dispensed droplet as it passes out of the opening of the nozzle to cause a temperature-based change on the temperature sensor 101a.

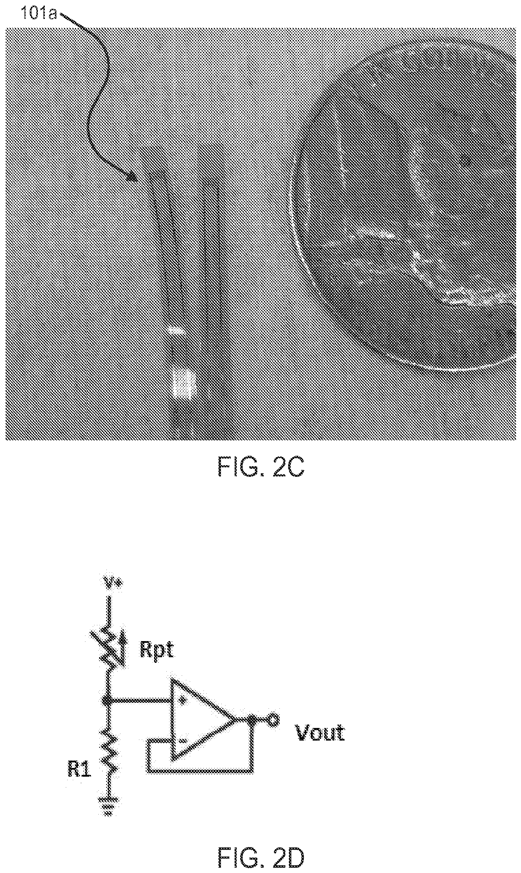

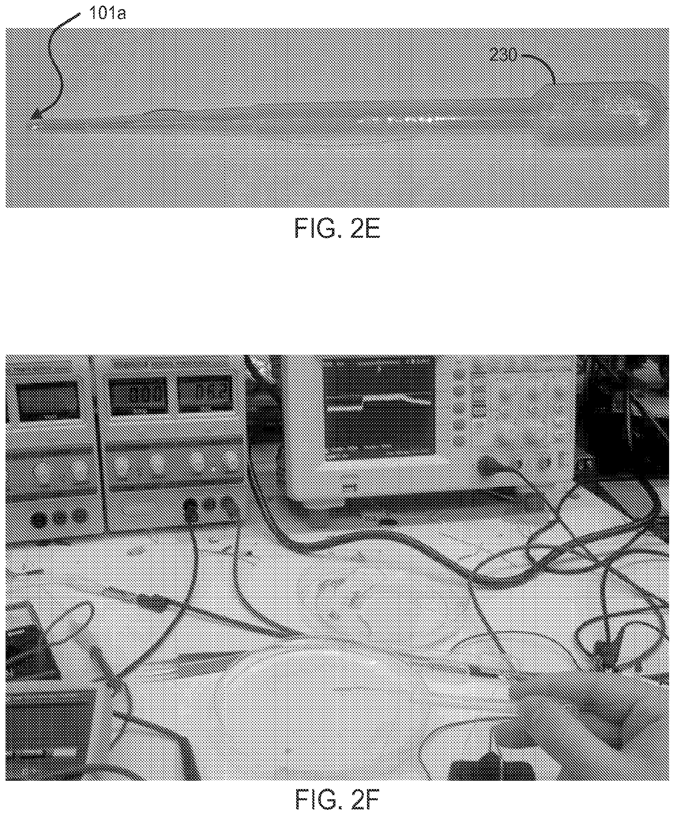

FIGS. 2C-2F show images and diagrams of an exemplary system to monitor dispensing of a droplet using the exemplary flexible sensor unit including a temperature dependent resistive flow sensor attached to a droplet dispenser. The images and diagrams of FIGS. 2C-2F show some aspects of the disclosed technology that may be included in various examples of the system 100. FIG. 2C shows an image of an example temperature dependent resistive flow sensor 101a of the flexible sensor unit 101, which is structured to include a platinum resistor electrically coupled to an electronic circuit. FIG. 2D shows a diagram of the electronic circuit and the platinum resistor to produce an output voltage based on a transduced occurrence detected by the sensor 101a, e.g., such as acquiring an eye drop at or near the tip of a container. FIG. 2E shows an image of an exemplary container 230, e.g., such as a droplet pipette, having attached to it an example of the system 100 including the temperature dependent resistive flow sensor 101a to detect the dispensing of a droplet from the pipette 230. FIG. 2F shows an image depicting the set-up of an exemplary implementation of the system 100 employed on the pipette 230, including the exemplary temperature dependent resistive flow sensor 101a wired to a remote signal processing and data processing unit.

### Optical Sensing

In some examples, the sensor unit 101 can include one or more optic flow sensors 101b. In such implementations, for example, a light emitting diode (LED) of the optical flow sensor unit 101b can be placed on the nozzle of the container 130 across from a photodetector of the optical flow sensor unit 101b. For example, as fluid passes through the nozzle, the refraction index changes in between the LED and photodetector. This translates to a change in the amount of light on the photodetector that is measured by a voltage change across the photodetector of the optic flow sensor unit 101b and processed to indicate that an amount of the liquid (e.g., such as a drop) was released from the container 130. For example, the measured signal (e.g., voltage change) can be amplified by the signal processing unit 105, and the processed signal subsequently sent to the data processing unit 110 (e.g., such as a microcontroller).

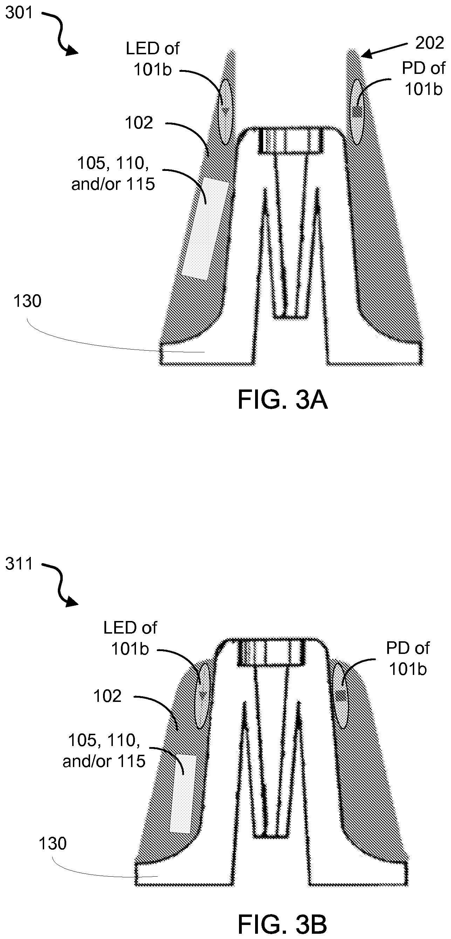

FIG. 3A shows a cross-sectional diagram of an exemplary optical data acquisition unit 301 of the disclosed technology unobtrusively attached to a droplet dispenser to detect the dispensing of a droplet from a container, e.g., in which the detected dispensing data can be processed for monitoring patient compliance. As shown in the diagram of FIG. 3A, the optical data acquisition unit 301 is attached to or integrated in the conformal substrate 102 of the system 100 that is attachable to the container 130, e.g., shown attached to the nozzle region of the container 130, and such that the system 100 is inconspicuously integrated with the container 130. In the example shown in FIG. 3A, the optical data acquisition unit 301 includes an optical sensor assembly 101b, e.g., including an LED placed on or embedded in the substrate 102 on an interior side of the substrate 102 within region 202, and a photodetector placed on or embedded in the substrate 102 on the opposite interior side of the substrate 102 within region 202. The optical sensor assembly 101b is configured on the conformal substrate 102 such that a dispensed droplet that passes through the interior passage of the region 202 will cause an optical-based change on the optical sensor assembly 101b, e.g., such as a refraction index change in between the LED and photodetector, where a change in the amount of light on the photodetector is transduced to a voltage change at the photodetector of the optical sensor assembly 101b. The optical sensor assembly 101b is in communication with the signal processing circuit 105, e.g., which can amplify and/or modulate the detected signal for data processing by the data acquisition unit 110, and/or for transmission to the device 120 by the wireless communication unit 115.

FIG. 3B shows a cross-sectional diagram of another exemplary optical data acquisition unit 311 of the disclosed technology unobtrusively attached to a droplet dispenser to detect the dispensing of a droplet from a container. As shown in the diagram of FIG. 3B, the optical data acquisition unit 311 is attached to or integrated in the conformal substrate 102 of the system 100 that is attachable to the nozzle of the container 130, such that the system 100 is inconspicuously integrated with the container 130. In the example shown in FIG. 3B, the optical data acquisition unit 311 includes the optical sensor assembly 101b configured on the conformal substrate 102 proximate the tip of the nozzle of the container 130 to detect a dispensed droplet as it passes out of the opening of the nozzle to cause an optical-based change on the optical sensor assembly 101b.

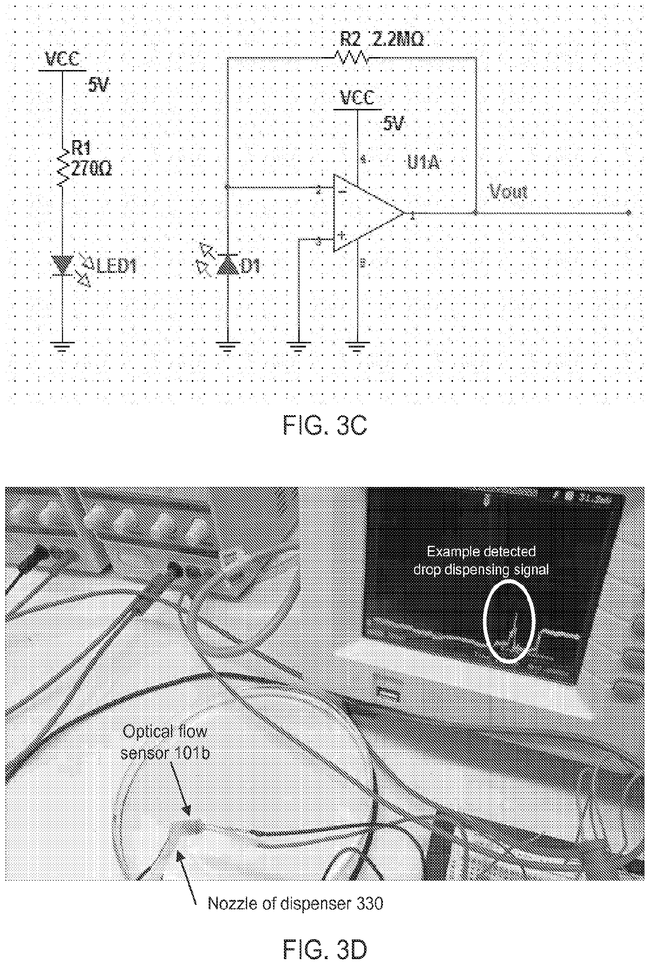

FIGS. 3C-3D show diagrams and images of exemplary flexible optical sensor systems to monitor dispensing of a droplet from a container nozzle. FIG. 3C shows a diagram of an exemplary circuit diagram to implement an exemplary amplification scheme to process the detected signal by the optical flow sensor. For example, as shown in the circuit diagram of FIG. 3C, the element labeled D1 represents the photodetector of the optical flow sensor and the element labeled LED1 represents the LED of the optical flow sensor. FIG. 3D shows an image depicting an exemplary set-up for implementation of the system 100 conformed on a droplet dispenser and including an optical data acquisition unit to optically detect the dispensing of a droplet form the dispenser. For example, as shown in FIG. 3D, flexible opto-electronics including an exemplary optical flow sensor 101b are attached at the base of a drop dispenser 330, e.g., on the outside of a nozzle, and an oscilloscope in the background of the image demonstrates how the exemplary circuit is capable of sensing the drop.

For example, in implementations of the system 100 including the temperature sensor 101a and/or the optical sensor 101b, the electronic components of the sensor unit 101 and/or other units of the system 100 can be integrated onto the outside of an existing bottle or container, as well as be embedded within the material of the bottle or container itself, e.g., during the manufacturing process of the bottle or container. For example, the flexible electronics can be integrated within an additive manufacturing framework where first the inner aspect of the nozzle, which touches the fluid, is made, followed by integration of flexible electronics, followed by fabrication of the outer nozzle. Similar procedures can also be employed in a manufacturing process to integrate the flexible electronics of system 100 into the container itself. For example, the exemplary flexible electronics of the system 100 can be embedded into the container 130 by using a 3D printing technique of the thin, flexible electronic components directly within or onto the materials that form the container or bottle. Exemplary advantages can be gained by integrating the flexible electronics into the container, including possibly eliminating the need for clinical studies of the bottle, as well as being totally indistinguishable from a normal bottle for the user. In such exemplary cases, wireless powering techniques can be used to power the integrated flexible electronics of the system 100. In other exemplary cases, the thin, flexible electronics can lie on the outside of the bottle for disposable use.

### Force/Pressure Sensing

The sensor unit 101 of the claimed device includes one or more sensors 101c to measure force and/or pressure, e.g., referred to as pressure sensors in this patent document. The pressure sensors 101c include a force sensitive resistor (FSR) or a strain gauge. In implementations of the system 100, the pressure sensor(s) 101c is placed over the body of the container 130 to detect squeezing of the container by a user. For example, a certain amount of pressure applied to (e.g., squeezed) the container 130 is required to release a certain amount of the liquid (e.g., such as a drop). The system 100 can determine the amount of pressure based on the acquired signals from the sensor unit 101 and determine if the detected amount of pressure is above or below a threshold, which can be used to infer the release of the drop, for example.

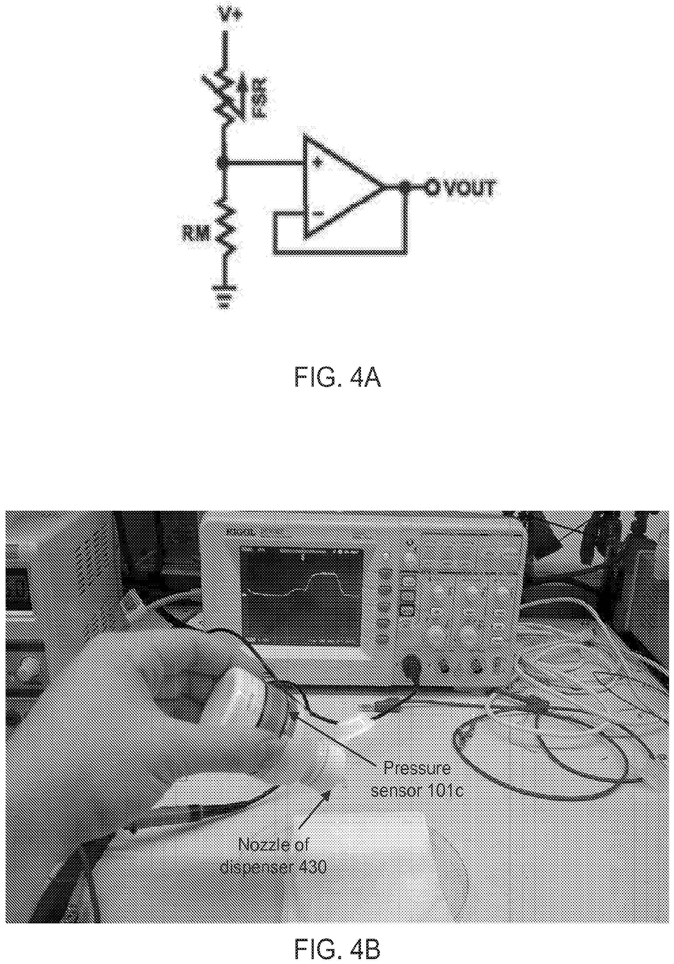

FIGS. 4A and 4B show diagrams and images of an exemplary system to monitor dispensing of a droplet using an exemplary flexible sensor unit including a pressure sensor attached to a droplet dispenser. FIG. 4A shows a diagram of an exemplary circuit, e.g., in an exemplary embodiment of the signal processing unit 105, to implement an exemplary amplification scheme using a force sensitive resistor (FSR) in the pressure sensor 101c of the system 100. FIG. 4B shows an image depicting an exemplary implementation of an exemplary force/pressure sensing embodiment of the system 100 attached to a squeezable container (e.g., a droplet dispenser) 430 including the pressure sensor unit 101c.

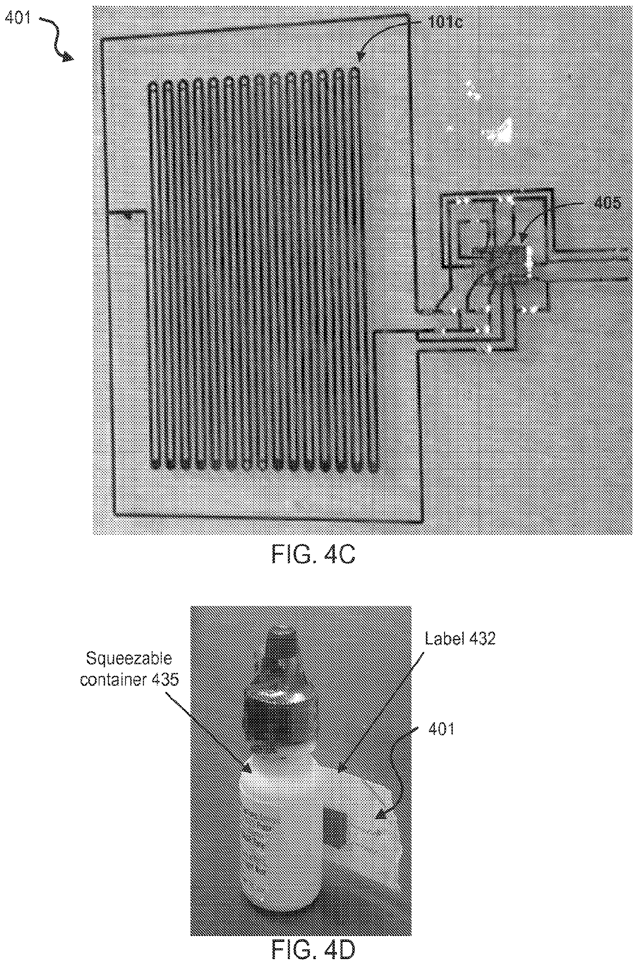

FIG. 4C shows an image of an exemplary force and/or pressure data acquisition unit 401 of the disclosed technology. The force/pressure data acquisition unit 401 includes a flexible pressure sensor 101c (e.g., FSR) electrically coupled to a flexible signal processing circuit 405. The force/pressure data acquisition unit 401 can be unobtrusively attached to a container, e.g., such as a fluid dispensing container, or blister pack container, or other type container, to detect the dispensing of a droplet from a fluid dispensing container, or to detect the piercing or tampering of a cover (e.g., lidding stock) over a blister in a blister pack container. For example, such detected signals can be processed and used for monitoring patient compliance of a medicine container.

FIG. 4D shows a diagram of the force/pressure data acquisition unit 401 integrated with a label 432 wrapped around a squeezable container 435 (e.g., droplet bottle) for a particular medicine (e.g., eye drops). For example, a flexible electronics device or system of the disclosed technology that includes the /pressure data acquisition unit 401 can be integrated with the label 432 in a separate manufacturing process from that of the medicine in the container 435. The force/pressure data acquisition unit 401 can be attached and conformed on the container 435 after the medicine is enclosed (sealed). In some implementations, for example, the force/pressure data acquisition unit 401 can be attached to the container 435 prior to application of the label 432. Whereas in some implementations, for example, the force/pressure data acquisition unit 401 can be incorporated into the label 432 be attached to the container 435.

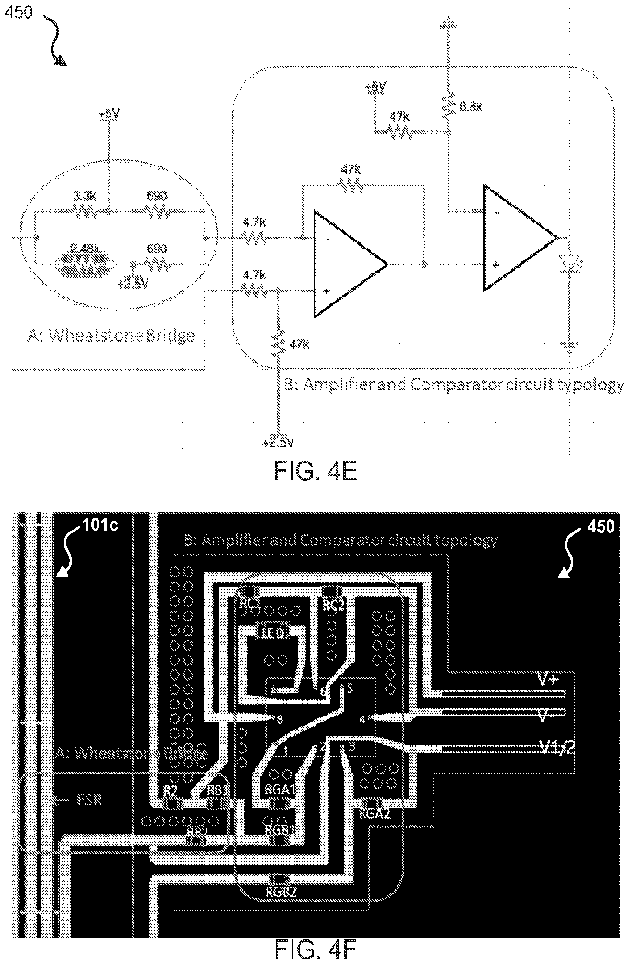

FIGS. 4E and 4F show diagrams of the exemplary flexible signal processing circuit 405 to process the detected force or pressure on the pressure sensor 101c, e.g., for monitoring use of a container. FIG. 4E shows a circuit diagram of an exemplary two stage signal processing circuit 405 including a Wheatstone bridge (e.g., stage A) and an amplifier and comparator circuit (e.g., stage B). The stage B includes an optical emitter (e.g., LED) to provide an optical signal indicative of detection of a force or pressure by the pressure sensor 101c. FIG. 4F shows a circuit diagram of the exemplary two stage signal processing circuit including a microchip including two operational amplifiers (Op Amps) in an 8-pinout configuration. For example, the exemplary two stage signal processing circuit 405 can be fabricated on the flexible and conformal substrate 102 to be bendable and/or stretchable and have a small footprint, e.g., in which the signal processing circuit 405 can be on the order of tens mm² (such as < 10 mm x < 10 mm dimensions).

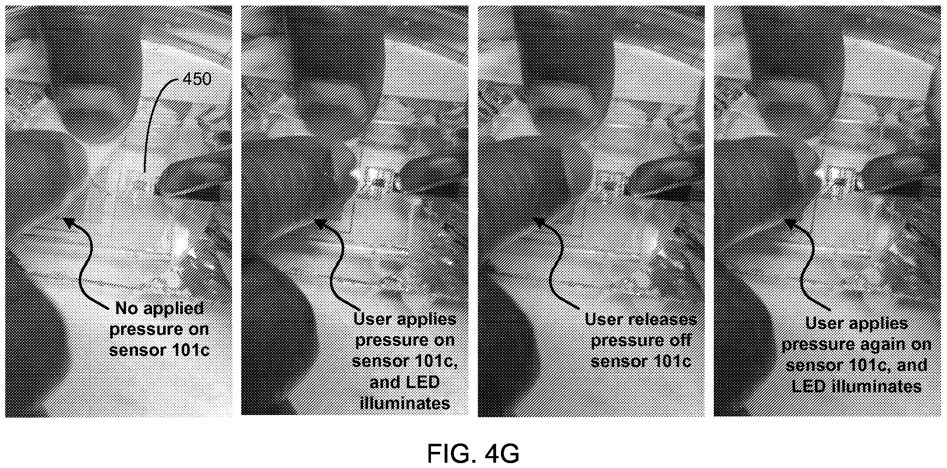

FIG. 4G shows a sequence of images depicting an implementation of the exemplary force/pressure data acquisition unit 401, including the optional LED to indicate that force or pressure is being applied. In the image sequence, at first (far-left image), a user is not applying any pressure onto the pressure sensor 101c (e.g., flexible FSR), which is indicated by the lack of light emitted by the signal processing circuit 450. Next (center-left image), the user applies pressure onto the pressure sensor 101c, which is indicated by a blue light emission by the LED of the signal processing circuit 450. Next (center-right image), the user releases pressure off of the pressure sensor 101c, which is indicated by the lack of light emitted by the signal processing circuit 450. Finally (far-right image), the user reapplies pressure onto the pressure sensor 101c, which is indicated by a blue light emission by the LED of the signal processing circuit 450.

In some implementations of the system 100, the force/pressure data acquisition unit 401 can be attached to a fully detachable or partially detachable lid of a container (e.g., medicine bottle) to monitor and detect the dispensing of the contents of the container by a user (e.g., detect an occurrence of when one or more pills contained in the medicine bottle are dispensed out of the medicine bottle).

### Position Sensing

In some examples, the sensor unit 101 can include one or more position sensors 101d. For example, the position sensors 101d can include a motion sensor such as a gyroscope or an accelerometer. Implementations of the system 100 can include using the position sensor 101d in conjunction with the pressure sensor 101c in the configuration of the sensor unit 101. In one exemplary implementation, the position sensor(s) 101d is placed on a location on the container 130 to detect its orientation, and the pressure sensor(s) 101c is placed over the body of the container 130 to detect squeezing of the container by a user. If the container 130 is detected to be at a certain angular position within a particular range and the pressure threshold is determined to have been crossed, then the system 100 can determine that it is likely for a drop to have been released. For example, this determination can eliminate a false positive when the bottle is squeezed without it being positioned to dispense drops.

In some implementations of the system 100, the position sensor 101d can be attached to a fully detachable or partially detachable lid of a container (e.g., medicine pill bottle) and a second position sensor 101d can be attached to the body of the container (e.g., the pill bottle itself) to monitor and detect dispensing events of the contents of the container by a user (e.g., detect an occurrence of when the lid is detached or partially detached and oriented different from the closed setting, and with reference to the orientation of the pill bottle body, in order to infer when one or more pills contained in the medicine bottle are dispensed out of the medicine bottle). Additionally, for example, the force/pressure data acquisition unit 401 can also be attached to the body of the container (e.g., medicine pill bottle) and/or the lid to provide additional data to that of the position sensors 101d to detect the dispensing of the contents from the container.

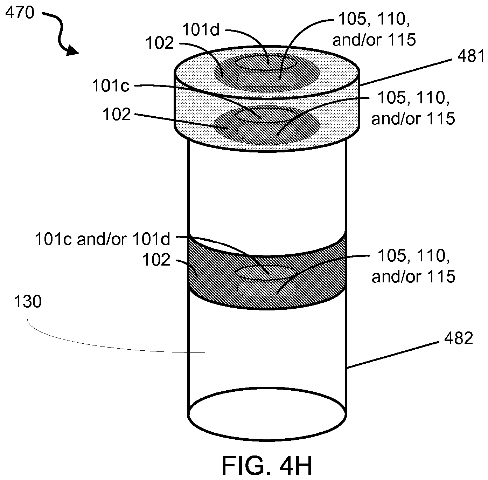

FIG. 4H shows an illustrative diagram of an exemplary pressure and position data acquisition unit 470 of the disclosed technology unobtrusively attached to a container having a detachable lid to detect the dispensing of the contents from the container, e.g., in which the detected dispensing data can be processed for monitoring patient compliance. As shown in the diagram of FIG. 4H, the position sensor 101c is attached to or integrated in the conformal substrate 102 of the system 100 that is attachable to a detachable or partially detachable lid 481 of the container 130, e.g., such that the system 100 is inconspicuously integrated with the lid 481. The pressure sensor 101d may alternatively or additionally be attached to or integrated in the conformal substrate 102 of the system 100 (e.g., either a separate substrate or the same substrate portion) that is attachable to the lid 481. An additional data acquisition unit 101 may be employed on the body 482 of the container 130. In the example shown in FIG. 4H, the pressure sensor 101c and/or the position sensor 101d is attached to or integrated in the conformal substrate 102 of the system 100 that is attachable to the body 482 of the container 130. The pressure and position data acquisition unit 470 is configured such that if a user squeezes the fully or partially detachable lid 481 to open the lid (and/or squeezes or changes the orientation of the body 482), the user's applied force or pressure and/or the change in orientation of the lid 481 (and/or the body 482) can be detected to indicate a dispensing event. The pressure and position data acquisition unit 470 is in communication with the signal processing circuit 105, e.g., which can amplify and/or modulate the detected signal for data processing by the data acquisition unit 110, and/or for transmission to the device 120 by the wireless communication unit 115. In addition, or alternatively, for example, the optical sensor 101b and/or the temperature sensor 101a can be configured at the opening of the body 482 to detect dispensing of the contents (e.g., fluid or pills) from the body 482 of the container 130.

The disclosed flexible electronics units of the system 100 can be fabricated using flexible electronics device fabrication techniques. The disclosed flexible electronic units of the system 100 can be embedded into the container 130 by using 3D printing methods that form the thin, flexible electronic components directly onto the materials that form the container or bottle. In one example, a base material can be created by 3D printing fabrication that produces an inner material layer that forms the interior portion of the bottle or container having an interior chamber that holds the fluid within. Subsequently, the exemplary flexible electronics units of the system 100 (e.g., such as the sensor unit 101, the signal processing unit 105, the data processing unit 110, and/or the wireless communications unit 115) can be 3D printed on the base material, e.g., at a particular location on the based material. For example, in some implementations, the exemplary flexible electronic units can be 3D printed on a conformal substrate including a mechanically flexible and an electrically insulative material that is attached to the inner material layer. Subsequently, an outer material can be created by 3D printing fabrication that produces an exterior material layer over the flexible electronics units of the system 100 to integrate them within the container or bottle structure. For example, the outer and base materials can be created with a shape, size, and/or geometry to form the container or bottle structure; or in some implementations, further material layers and structures can be added (e.g., using 3D printing) to produce the overall structure of the container with the integrated system 100 within the container structure. In some device fabrication implementations, for example, the disclosed flexible electronics units of the system 100 can be fabricated and embedded into the container, e.g., using injection molding techniques. For example, the flexible electronics can be placed inside a mold and an insulating polymer material that is compatible can be injected into the mold.

### Exemplary Software Application

Exemplary feedback schemes to assist in patient compliance of using a medication by a bottle implementing the disclosed systems are described. In one example, using a wireless connection (e.g., Bluetooth) between a user's smartphone as an example of the mobile device 120 and the container 130 having the system 100 attached, the system 100 can log the release timing of a drop from the container 130 on the smartphone and issue reminders for the patient to take their medication and/or indicate when the patient forgets.

The disclosed technology can include a software application resident on the user's mobile device 120 and/or computing device(s) of the same or other users, e.g., such as caregivers to the patient user, to manage information associated with the detected use of the container by the user (e.g., detected droplet dispensing, container cap removal, or puncture/peeling of cover over blister). For example, the software application can provide an interface for a user (e.g., the patient, caregiver, or other type of user) to access information about their usage of the contents inside the container having the system 100 applied to it, e.g., providing temporal usage information on the day and time that the contents was released from the container (e.g., a droplet, pill, or dosage of a medication was taken by the patient). For example, the information provided via the software application can include a percentage of medicine taken (e.g., percentage of drops taken) within a specified time window, e.g., which can be important for clinicians or other care providers to determine if the effect of a medical condition (e.g., an eye condition) worsening is due to infeasibility of the current intervention or due to lack of compliance. The software application can allow tracking of when an amount of medicine was taken (e.g., droplet dispensed, or pill removed from a container vial or blister pack), and the software application can provide alerts or messages to users (e.g., the patient, caregiver, or other intended user) to notify the patient user to take the medicine and/or notify the caregiver that the user has or has not taken the medicine (e.g., for a particular time or time period). The software application can provide such alerts and notifications by sending displaying a notification on the display screen of the mobile device 120, sending a text message to the user(s), and/or transmitting command data from the mobile device 120 to the system 100 on the container 130 to provide an alert signal (e.g., light an LED (e.g., blinking or a color coded illumination), sound an audio alarm via a speaker, and/or cause a haptic response).

The software application can process the data received from the system 100 to determine if multiple doses (e.g., drops) were taken in a single suggested dosing interval. Such information can be used by caregivers to understand other challenges for the patient in compliance with the prescribed regimen. Moreover, such dose tracking (e.g., droplet dispensing, or pill dispensing) information can be processed by the software application to determine how much medicine may be remaining in the medicine container. As such, the software application can estimate when the patient should be finished with the medicine in that particular container (e.g., when the container will be emptied), and act on the estimated information. For example, the software application can provide notification to the patient or caregiver to contact the pharmacy or physician for refill of the medication, or the software application can automatically inform the pharmacy or physician to refill the medication. By accurately tracking the usage (e.g., droplets), the software application can mitigate risks to prevent the patient from being out of supply of their medication and not miss any dosage.

For example, many eye medications include droplet products that are for the elderly and aging population, who may suffer from diminishing manual dexterity. The software application can provide information to caregivers on the patient's ability to squeeze and/or aim the medicine container including the system 100 having the pressure sensor and/or position sensor units. Moreover, some patients (e.g., including elderly patients) may be taking one or more other medications for conditions that may be associated with diminishing dexterity (e.g., Parkinson's Disease). For example, suppose a patient with Parkinson's Disease and an eye condition requiring droplet medication takes the Parkinson's Disease medicine on certain days or certain times per day. The tracking of the patient's ability to manage the squeezing and aiming of the eye medication container having the system 100 may play a role in providing useful information to a caregiver regarding the patient's other condition, e.g., Parkinson's Disease. The software application can be used to integrate information across multiple different medication types and then help the understanding of their efficacy. For example, a container vial with a detachable lid or a blister pack having the system 100 can provide information to the caregiver via the software application that characterizes when the patient takes a swallowable pill whose effect can correlate with the effect of the droplet medication being tracked - this information is actionable. The information about the multiple medications is important to be displayed and statistically quantified for the purpose of improved decision support. By integrating that data and correlating dosage across suggested dosage, within a specific medication as well as across, a caregiver can better understand the efficacy of the medication.

The software application can process the data received from the system 100 to monitor security and safety related issues associated with the medication contained in the container 130. For example, software application can process the data received from the system 100 to determine if multiple doses (e.g., drops or pills) were taken in a particular dosing interval to indicate potential abuse of the medication. For example, the software application can include data processing methods that identify potential substance addiction behavior. For example, if the software application can process the detection data to identify instances and/or patterns of use that are inconsistent with a predetermined pattern of use (e.g., prescription is for one pill or droplet per day, to be taken at night). Such identified instances can be reported to the caregiver or other entities. Similarly, the software application can provide location data associated with the detection of the dispensing occurrence(s) and/or quantity of dispensing. For example, in some examples, the data processing unit 110 of the system 100 can include a location positioning unit to determine the location of the system 100. For example, in some implementations, the location positioning unit can include a GPS device. In some implementations, for example, the system 100 can include location processing techniques to determine an approximate location of the system based on communications between the wireless transmitting unit 115 and remote devices (e.g., WiFi transmitter/receiver). In some implementations, for example, the software application can notify certain entities (e.g., the patient, caregivers, etc.) if one or more dispensing events have occurred based on data stored in memory of the data processing unit 110 without associated communications of those dispensing events from the system 100 to the user's mobile device 120, e.g., where such instances could be indicative of an improper use of the medicine in the container 130.

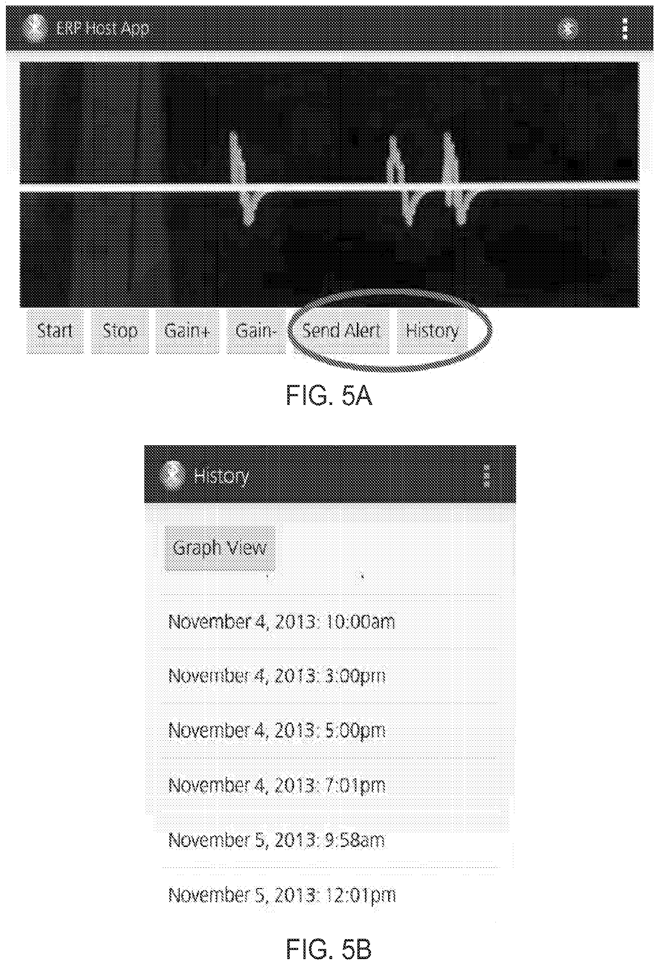

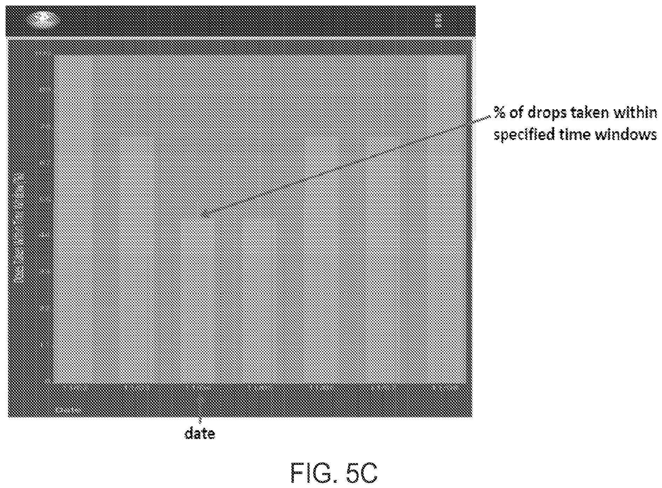

FIG. 5A shows a screen shot diagram of an example user interface of an exemplary software application of the disclosed technology resident on the mobile device 120, showing a time response of droplet administration detected by the system 100 on an example droplet dispenser. FIG. 5B shows a screen shot diagram of an example interface showing a time history of the medicine administration (e.g., dispensed droplets) performed by a user detected by the disclosed flexible electronics devices. FIG. 5C shows a screen shot diagram of an example interface showing a percentage time history of medicine administration in a given time period performed by the user detected by the disclosed technology.

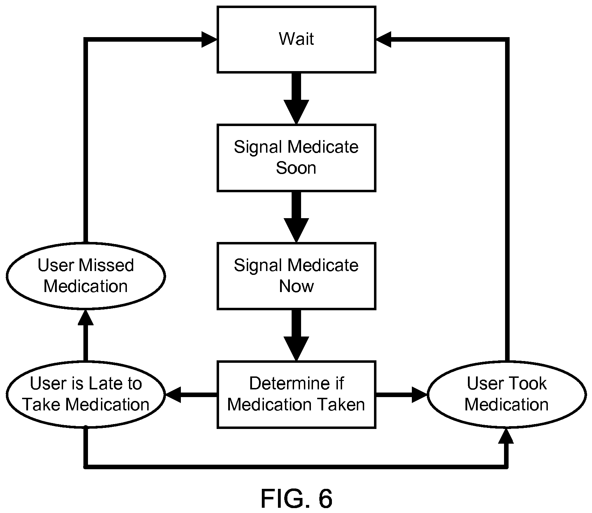

In another example, an internal timer on the bottle can be set for an optimal dosage time. At a set interval prior to this time, the bottle illuminates to indicate that it is almost time to medicate. During the optimal medication window, the bottle changes states (e.g., a change in color or blinking in the lights) to indicate that the medicine should be taken. If the medicine was taken, for example, the bottle changes states again to indicate the medicine was taken. If the medicine was not taken within the optimal medication window, for example, the bottle will change state to indicate that the dose was missed or late. Each state can be logged to internal memory of the data processing unit 110 or a memory unit of a remote computer and extracted later to check patient compliance. This exemplary second feedback scheme is depicted in the flow diagram of FIG. 6. FIG. 6 shows a flow diagram of an exemplary feedback method to facilitate patient compliance in taking a medication using a bottle incorporating an exemplary flexible electronic monitoring system of the disclosed technology.

The disclosed approach uses ultra-thin flexible electronics that remain unobtrusive to a user by inserting into or wrapping them around a container, e.g., such as a bottle. In some examples, the flexible electronic components that are used in the various examples of the system 100 can be produced by using bare die chips integrated into flexible polymer substrates (e.g., polyimide or elastomers). For example, such techniques can take existing small chip components and integrate into a flexible electronics solution. Such fabrication techniques can provide flexible electronics systems and devices, as described in this patent document, to allow for an ergonomic sensing container or bottle that can be utilized in a variety of ways, e.g., including monitoring and intervening in patient compliance for use of a medication or other substance.

### Blister Pack Examples

In some aspects, the disclosed flexible electronic sensor and wireless communication technology can integrate directly into blister pack configurations of medical dispensers, or other type of dispensers, to infer the release of the contents (e.g., medication) from the blister pack. The disclosed flexible electronics devices and systems combine sensing logic and wireless communication capabilities to facilitate patient compliance through monitoring of medication dispensing in time. Such flexible electronics devices and systems of the disclosed technology can be incorporated into existing non-electronic blister packs through additive post-processing, in which the flexible electronics system can be built independently and attached directly to the frangible backing of a blister pack in its current off-the-shelf state, thus providing a simple 'two-step' manufacturing method of electronic blister packs.

Existing patient compliance systems are obtrusive and typically require manufacturing of new medical dispensers to enable monitoring of patient compliance. Moreover, these conventional systems can require burdensome procedures to extract monitored information (e.g., such as non-wireless or non-automated measures), which may even cause non-compliance. In contrast, the disclosed flexible electronic sensor and wireless communication technology includes designs that can be produced in an additive manner and that have no obtrusive features that could interfere with patient compliance.

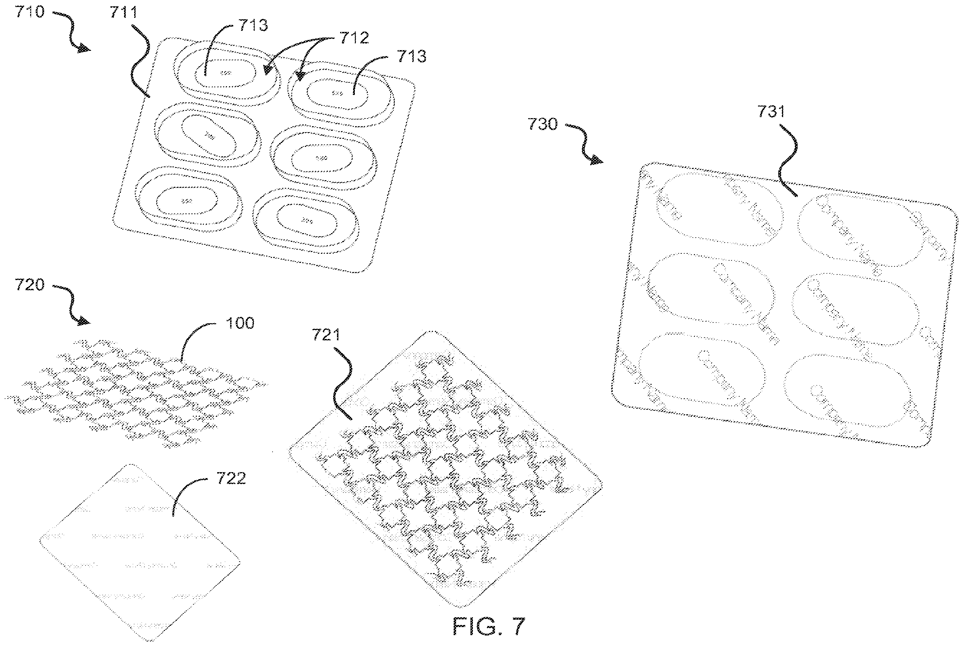

In some examples, the disclosed technology includes an example of the system 100 included in a frangible sheet that can be attached to a blister pack container pre-filled with medicine, where the frangible sheet and coupled system 100 are attached to the blister pack container after the medicine is sealed and secured, and in which the system 100 can unobtrusively monitor the release of each medication from the blister pack container. FIG. 7 shows an illustrative diagram of a method to produce a blister pack container including the system 100.

As shown in FIG. 7, process 710 includes the fabrication of a blister pack container 711 including blister troughs 712 to contain the manufactured medication 715, e.g., pills already dispensed within the blister pack container base 711. The container base 711 can include a sealant layer attached over the blister troughs 712 that enclose and seal the medication 713 inside (e.g., such as a frangible paper backing laminated onto a foil backing, much like a label on existing commercial blister pack containers). Process 720 includes the fabrication of a frangible backing 721 for the blister pack container 711 that includes the system 100 (e.g., such as the system 100 including the force/pressure data acquisition unit 401) on a substrate 722 of the frangible backing 721. The frangible backing 721 includes a mounting side that includes the system 100 and an adhesive to attach to the pre-filled and pre-sealed blister pack container base 711. In some implementations, for example, the frangible backing 721 can include labeling or messaging on the outer side of the frangible backing 721. The process 710 and the process 720 can be implemented separately and are independent from each other. Process 730 includes the combination of the medication-filled blister pack container 711 and the frangible backing 721 including the system 100 to form a smart blister pack medicine container 731 capable of patient compliance. For example, the process 730 includes attaching the mounting side of the frangible backing 721 to the sealed layer of the blister pack container 711. In some implementations, for example, the attachment of the backing 721 to the blister pack 711 can be performed by laminating or other attachment processes. The flexible electronic device 100 is not seen, but is operable to detect peeling off of or puncture through of the frangible backing 721 over each one of the several blister troughs 712.

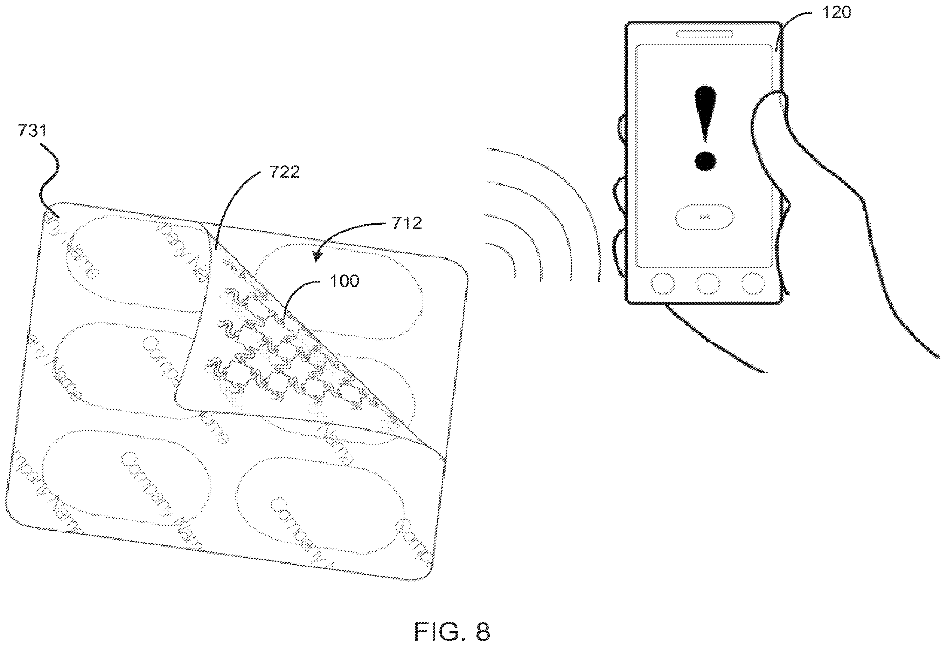

FIG. 8 shows an illustrative diagram of a patient using the smart blister pack medicine container 731 to peel off a portion of the frangible backing 721 (e.g., to access a pill pre-sealed in the blister trough 712), in which the system 100 is able to detect the user's peeling of the frangible backing 721 to transmit the detected data to the user's mobile device 120.

While several of the disclosed examples that are described in this patent document are primarily based on monitoring the dispensing of medicine from a medicine container (e.g., such as droplet dispenser, pill bottle with detachable lid, and blister pack) to facilitate understanding of the underlying concepts of the disclosed technology, it is understood that the disclosed devices, systems, and methods can also include monitoring of other contents and substances in similar type containers, which include, but are not limited to, foods, drinks and food-related products (e.g., vitamins, nutrient supplements, gum, candy), nicotine delivery products (e.g., nicotine gum), and non-consumable products.

### Examples

The following examples the present technology are also disclosed.

In one example of the present technology (example 1), a device for monitoring patient compliance of a medication includes a substrate that is attachable and conformable to a container containing medicine; a data acquisition unit including a sensor and a signal processing circuit formed on the substrate, in which the sensor is operable to transduce an occurrence of the medicine dispensing from the container into an electrical signal, and the signal processing circuit is operable to amplify the electrical signal; a data processing unit including a processor and a memory formed on the substrate and in communication with the data acquisition unit to process the amplified electrical signal as data; and a communications unit formed on the substrate and in communication with the data processing unit to wirelessly transmit the processed data to a remote device.

Example 2 includes the device as in example 1, in which the substrate includes a mechanically flexible and an electrically insulative material.

Example 3 includes the device as in example 1, in which the device is capable to inconspicuously attach to the container.

Example 4 includes the device as in example 1, in which the container includes a droplet bottle, a blister pack, or a bottle with a detachable lid.

Example 5 includes the device as in example 1, in which the data processing unit is configured to determine an amount of the medicine dispensed from the container based on the electrical signal.

Example 6 includes the device as in example 1, in which the processed data indicates a time at which the determined dispensing event occurred.

Example 7 includes the device as in example 6, in which the data processing unit is configured to determine if the time at which the determined dispensing event occurred is before or after a predetermined time.

Example 8 includes the device as in example 7, in which the data processing unit is configured to produce a message indicating the dispensing event was early or late based on the predetermined time.

Example 9 includes the device as in example 1, in which the sensor includes one or more of a temperature sensor, an optical sensor, a pressure sensor, or a position sensor.

Example 10 includes the device as in example 9, in which the medicine includes a fluid, and the substrate is attached and conformed at or proximate to a nozzle of the container.

Example 11 includes the device as in example 10, in which the temperature sensor includes a temperature dependent resistive flow sensor operable to produce the electrical signal by detecting a temperature fluctuation when the fluid passes out of the nozzle.

Example 12 includes the device as in example 10, in which the optical sensor includes a light emitting diode (LED) positioned at one end of the nozzle and a photodetector positioned at an opposing end of the nozzle to receive light emitted by the LED, in which the optical sensor is operable to produce the electrical signal by detecting, at the photodetector, a change in a refraction index of the emitted light caused when the fluid passes out of the nozzle.

Example 13 includes the device as in example 9, in which the medicine includes a fluid, and the substrate is attached and conformed to the body of the container.

Example 14 includes the device as in example 13, in which the pressure sensor includes a force sensitive resistor (FSR) or a strain gauge, in which the pressure sensor is operable to produce the electrical signal corresponding to an amount of pressure change caused by squeezing the body of the container.

Example 15 includes the device as in example 14, in which the data processing unit is operable to determine if the detected amount of pressure is above or below a threshold value to determine the occurrence of the dispensing event.

Example 16 includes the device as in example 9, in which the position sensor includes a gyroscope or an accelerometer, in which the position sensor is operable to produce the electrical signal based on a change in orientation of the container detected by the position sensor.

Example 17 includes the device as in example 1, further including an LED electrically coupled to the signal processing circuit and operable to emit a light when the sensor detects the occurrence of the medicine dispensing from the container.

Example 18 includes the device as in example 1, further including an alert unit including one or more of an LED, a speaker, or a vibrator in communication with the data processing unit to generate an alert signal from the container.

Example 19 includes the device as in example 18, in which the data processing unit is operable to determine compliance of a user of the container for taking the medicine according to a schedule of use for the user, and to produce a command signal to actuate the alert unit to generate the alert signal when the dispensing event was early or late based on the schedule of use.

In another example of the present technology (example 20), a method for monitoring dispensing of medicine from a container includes detecting a signal by a sensor on a conformal substrate attached and conformed to a container containing medicine; and processing the detected signal to produce data indicative of an occurrence or a non-occurrence of the medicine dispensed from the container.

Example 21 includes the method as in example 20, in which the conformal substrate includes a mechanically flexible and an electrically insulative material, and in which the sensor includes one or more of a temperature dependent resistive flow sensor, an optic flow sensor, a pressure sensor, or a position sensor.

Example 22 includes the method as in example 20, in which the detecting includes transducing events corresponding to the medicine dispensing and not dispensing from the container into distinguishable electrical signals, and amplifying the electrical signals.

Example 23 includes the method as in example 20, in which the processing includes determining an amount of the medicine dispensed from the container based on the detected signal.

Example 24 includes the method as in example 20, in which the processing includes associating a time value with a determined occurrence.

Example 25 includes the method as in example 24, in which the processing includes determining if the time value associated with the determined occurrence is before or after a predetermined time.

Example 26 includes the method as in example 20, in which the processing is performed by a data processing unit on the conformal substrate.

Example 27 includes the method as in example 26, further including wirelessly transmitting the processed data to a remote device.

Example 28 includes the method as in example 27, further including producing an alert corresponding to a message indicating a dispensing event is upcoming, a dispensing event has occurred, or a dispensing event has been missed.

Example 29 includes the method as in example 20, further including determining compliance of a user using the medicine according to a particular schedule of use for the user.

Example 30 includes the method as in example 29, further including generating a message indicating the dispensing event was early or late based on the predetermined time.

Example 31 includes the method as in example 20, in which the container includes a droplet bottle, a blister pack, or a bottle with a detachable lid.

In another example of the present technology (example 32), a device for monitoring dispensing of a fluid from a container includes a data acquisition unit including a sensor and a signal processing circuit formed in a material structure of a container capable of storing and dispensing a fluid, in which the sensor transduces an occurrence of the fluid dispensing from the container into an electrical signal, and the signal processing circuit amplifies the electrical signal; a data processing unit including a processor and a memory unit, the data processing unit formed in the material structure of the container and in communication with the data acquisition unit to process the amplified electrical signal as data to determine a dispensing event of the fluid from the container; and a communications unit formed in the material structure of the container and in communication with the data processing unit to wirelessly transmit the processed data to a remote device.

Example 33 includes the device as in example 32, in which the data acquisition unit, the data processing unit, and the communications unit are formed in the material structure of the container by a 3D printing process.

Example 34 includes the device as in example 32, in which the container includes a droplet bottle or a bottle with a detachable lid.

Example 35 includes the device as in example 32, in which the sensor includes one or more of a temperature sensor, an optical sensor, a pressure sensor, or a position sensor.

Example 36 includes the device as in example 32, in which the sensor is configured at or proximate to a nozzle of the container in the material structure.

Example 37 includes the device as in example 36, in which the temperature sensor includes a temperature dependent resistive flow sensor operable to produce the electrical signal by detecting a temperature fluctuation when the fluid passes out of the nozzle.

Example 38 includes the device as in example 36, in which the optical sensor includes a light emitting diode (LED) positioned at one end of the nozzle and a photodetector positioned at an opposing end of the nozzle to receive light emitted by the LED, in which the optical sensor is operable to produce the electrical signal by detecting, at the photodetector, a change in a refraction index of the emitted light caused when the fluid passes out of the nozzle.

Example 39 includes the device as in example 32, in which the sensor is configured in the body of the container in the material structure.

Example 40 includes the device as in example 39, in which the pressure sensor includes a force sensitive resistor (FSR) or a strain gauge, in which the pressure sensor is operable to produce the electrical signal corresponding to an amount of pressure change caused by squeezing the body of the container.

Example 41 includes the device as in example 39, in which the position sensor includes a gyroscope or an accelerometer, in which the position sensor is operable to produce the electrical signal based on a change in orientation of the container detected by the position sensor.

In another example of the present technology (example 42), a device for monitoring dispensing from a container includes a substrate that is attachable and conformable to a container; a data acquisition unit including a sensor and a signal processing circuit formed on the substrate, in which the sensor is operable to transduce an occurrence of a content contained in the container dispensing from the container into an electrical signal, and the signal processing circuit is operable to amplify the electrical signal; and a communications unit formed on the substrate and in communication with the signal processing circuit to wirelessly transmit the amplified electrical signal to a remote device.

Example 43 includes the device as in example 42, in which the substrate includes a mechanically flexible and an electrically insulative material.

Example 44 includes the device as in example 42, further including a data processing unit including a processor and a memory formed on the substrate, in which the data processing unit is in communication with the data acquisition unit to process the amplified electrical signal as data, and in communication with the communications unit to provide the processed data to be wirelessly transmitted to the remote device.

Example 45 includes the device as in example 42, in which the sensor includes a temperature sensor positioned at an opening of the container and operable to produce the electrical signal by detecting a temperature fluctuation when the content passes out of the opening of the container.

Example 46 includes the device as in example 42, in which the sensor includes an optical sensor including a light emitting diode (LED) positioned at one end of an opening of the container and a photodetector positioned at an opposing end of the opening to receive light emitted by the LED, in which the optical sensor is operable to produce the electrical signal by detecting, at the photodetector, a change in the emitted light caused when the contents passes out of the opening of the container.

Example 47 includes the device as in example 42, in which the sensor includes a force or pressure sensor operable to produce the electrical signal corresponding to an amount of pressure change applied on a portion of the container where the sensor is attached.

Example 48 includes the device as in example 42, in which the sensor includes a position sensor operable to produce the electrical signal based on a change in orientation of the container detected by the position sensor.

The additional following examples are also disclosed.

In one example of the present technology (example A1), a method for monitoring dispensing of a fluid from a container includes detecting a signal by a sensor on a conformal substrate attached and conformed to a container containing a fluid; processing the detected signal as data using a processing unit on the conformal substrate, the processing including determining occurrences and non-occurrences of the fluid dispensed from the container, and associating a time value with the determined occurrence; and wirelessly transmitting the processed data to a remote device.

Example A2 includes the method as in example A1, in which the detecting includes transducing events corresponding to the fluid dispensing and not dispensing from the container into distinguishable electrical signals, and amplifying the electrical signals.

Example A3 includes the method as in example A1, in which the processing further includes determining an amount of the fluid dispensed from the container based on the detected signal.

Example A4 includes the method as in example A1, in which the processing further includes determining if the time value associated with the determined occurrence is before or after a predetermined time.

Example A5 includes the method as in example A4, further including producing raw message data corresponding to a message indicating the occurrence was early or late based on the predetermined time; and transmitting the message data to the remote device.

Example A6 includes the method as in example A1, further including determining compliance of a user using the fluid according to a particular schedule of use.

Example A7 includes the method as in example A1 or A6, in which the fluid includes a medicine.

Example A8 includes the method as in example A1 or A7, in which the container includes an eye drop bottle.

Example A9 includes the method as in example A1, in which the conformal substrate includes a mechanically flexible and an electrically insulative material.

Example A10 includes the method as in example A1, in which the sensor includes one or more of a temperature dependent resistive flow sensor, an optic flow sensor, a pressure sensor, or a position sensor.

In another example of the present technology (example A11), a system for monitoring dispensing of a fluid from a container includes a conformal substrate including a mechanically flexible and an electrically insulative material, the conformal substrate attached and conformed to a container containing a fluid; a data acquisition unit including a sensor and a signal processing circuit formed on the conformal substrate, in which the sensor transduces an occurrence of the fluid dispensing from the container into an electrical signal, and the signal processing circuit amplifies the electrical signal; a data processing unit including a processor and a memory unit, the data processing unit formed on the conformal substrate and in communication with the data acquisition unit to process the amplified electrical signal as data to determine a dispensing event of the fluid from the container; and a communications unit formed on the conformal substrate and in communication with the data processing unit to wirelessly transmit the processed data to a remote device.

Example A12 includes the system as in example A11, in which the data processing unit is configured to determine an amount of the fluid dispensed from the container based on the electrical signal.

Example A13 includes the system as in example A1, in which the processed data indicates a time at which the determined dispensing event occurred.

Example A14 includes the system as in example A13, in which the data processing unit is configured to determine if the time at which the determined dispensing event occurred is before or after a predetermined time.

Example A15 includes the system as in example A14, in which the data processing unit is configured to produce a message indicating the dispensing event was early or late based on the predetermined time.

Example A16 includes the system as in example A11 or A15, in which the fluid includes a medicine.

Example A17 includes the system as in example A11 or A16, in which the container includes an eye drop bottle.

Example A18 includes the system as in example A11, in which the sensor includes one or more of a temperature dependent resistive flow sensor, an optic flow sensor, a pressure sensor, or a position sensor.

Example A19 includes the system as in example A18, in which the conformal substrate is attached and conformed on or proximate to a nozzle of the container.

Example A20 includes the system as in example A19, in which the temperature dependent resistive flow sensor produces the electrical signal by detecting a temperature fluctuation when the fluid passes out of the nozzle.

Example A21 includes the system as in example A19, in which the optic flow sensor includes a light emitting diode (LED) positioned at one end of the nozzle and a photodetector positioned at an opposing end of the nozzle operable to receive light emitted by the LED, in which the optic flow sensor produces the electrical signal by detecting, at the photodetector, a change in the refraction index of the emitted light caused when the fluid passes over the nozzle.

Example A22 includes the system as in the example A21, further including an optically emitting element (e.g., such as an LED) in communication with the data processing unit, the optically emitting element operable to emit light at a direction and/or intensity to illuminate the container, in which the data processing unit is configured to control the optically emitting element to emit the light to provide an alert signal indicating to the user that the user should dispense the fluid from the container.

Example A23 includes the system as in example A18, in which the conformal substrate is attached and conformed to the body of the container.

Example A24 includes the system as in example A23, in which the pressure sensor includes a force sensitive resistor (FSR) or a strain gauge, in which the pressure sensor produces the electrical signal corresponding to an amount of pressure change caused by squeezing the body of the container, and in which the processing unit determines if the detected amount of pressure is above or below a threshold value to determine the occurrence of the dispensing event.

Example A25 includes the system as in example A24, in which the position sensor includes a gyroscope or an accelerometer, in which the position sensor produces the electrical signal based on a change in orientation of the container detected by the position sensor.

In another example of the present technology (example A26), a system for monitoring dispensing of a fluid from a container includes a data acquisition unit including a sensor and a signal processing circuit formed in a material structure of a container capable of storing and dispensing a fluid, in which the sensor transduces an occurrence of the fluid dispensing from the container into an electrical signal, and the signal processing circuit amplifies the electrical signal; a data processing unit including a processor and a memory unit, the data processing unit formed in the material structure of the container and in communication with the data acquisition unit to process the amplified electrical signal as data to determine a dispensing event of the fluid from the container; and a communications unit formed in the material structure of the container and in communication with the data processing unit to wirelessly transmit the processed data to a remote device.

Example A27 includes the system as in example A26, in which the data acquisition unit, the data processing unit, and the communications unit are formed in the material structure of the container by a 3D printing process.

Implementations of the subject matter and the functional operations described in this patent document can be implemented in various systems, digital electronic circuitry, or in computer software, firmware, or hardware, including the structures disclosed in this specification, or in combinations of one or more of them. Implementations of the subject matter described in this specification can be implemented as one or more computer program products, i.e., one or more modules of computer program instructions encoded on a tangible and non-transitory computer readable medium for execution by, or to control the operation of, data processing apparatus. The computer readable medium can be a machine-readable storage device, a machine-readable storage substrate, a memory device, a composition of matter affecting a machine-readable propagated signal, or a combination of one or more of them. The term "data processing apparatus" encompasses all apparatus, devices, and machines for processing data, including by way of example a programmable processor, a computer, or multiple processors or computers. The apparatus can include, in addition to hardware, code that creates an execution environment for the computer program in question, e.g., code that constitutes processor firmware, a protocol stack, a database management system, an operating system, or a combination of one or more of them.

A computer program (also known as a program, software, software application, script, or code) can be written in any form of programming language, including compiled or interpreted languages, and it can be deployed in any form, including as a stand-alone program or as a module, component, subroutine, or other unit suitable for use in a computing environment. A computer program does not necessarily correspond to a file in a file system. A program can be stored in a portion of a file that holds other programs or data (e.g., one or more scripts stored in a markup language document), in a single file dedicated to the program in question, or in multiple coordinated files (e.g., files that store one or more modules, sub programs, or portions of code). A computer program can be deployed to be executed on one computer or on multiple computers that are located at one site or distributed across multiple sites and interconnected by a communication network.

The processes and logic flows described in this specification can be performed by one or more programmable processors executing one or more computer programs to perform functions by operating on input data and generating output. The processes and logic flows can also be performed by, and apparatus can also be implemented as, special purpose logic circuitry, e.g., an FPGA (field programmable gate array) or an ASIC (application specific integrated circuit).

Processors suitable for the execution of a computer program include, by way of example, both general and special purpose microprocessors, and any one or more processors of any kind of digital computer. Generally, a processor will receive instructions and data from a read only memory or a random access memory or both. The essential elements of a computer are a processor for performing instructions and one or more memory devices for storing instructions and data. Generally, a computer will also include, or be operatively coupled to receive data from or transfer data to, or both, one or more mass storage devices for storing data, e.g., magnetic, magneto optical disks, or optical disks. However, a computer need not have such devices. Computer readable media suitable for storing computer program instructions and data include all forms of nonvolatile memory, media and memory devices, including by way of example semiconductor memory devices, e.g., EPROM, EEPROM, and flash memory devices. The processor and the memory can be supplemented by, or incorporated in, special purpose logic circuitry.

While this patent document contains many specifics, these should not be construed as limitations on the scope of the claims, but rather as descriptions of features that may be specific to particular examples of the disclosure. Certain features that are described in this patent document in the context of separate examples can also be implemented in combination in a single example. Conversely, various features that are described in the context of a single example can also be implemented in multiple examples separately or in any suitable subcombination..

Similarly, while operations are depicted in the drawings in a particular order, this should not be understood as requiring that such operations be performed in the particular order shown or in sequential order, or that all illustrated operations be performed, to achieve desirable results. Moreover, the separation of various system components in the examples described in this patent document should not be understood as requiring such separation in all examples.

Only a few implementations and examples are described and other implementations, enhancements and variations can be made based on what is described and illustrated in this patent document.

## Claims

1. A device (100) for monitoring patient compliance of a medication, comprising:
a substrate (102) suitable for attaching and conforming to a bottle container (130) containing medicine including a fluid, wherein the substrate includes a mechanically flexible and an electrically insulative material;
a data acquisition unit including a sensor (101) and a signal processing circuit (105) formed on the substrate, wherein the sensor is operable, when the device is attached to a body of the bottle container, to transduce an occurrence of the medicine dispensing from the bottle container into an electrical signal, and the signal processing circuit is operable to amplify the electrical signal, wherein the sensor (101) includes a pressure sensor (101c);
a data processing unit (110) including a processor (111) and a memory (114) formed on the substrate and in communication with the data acquisition unit to process the amplified electrical signal as data; and
a communications unit (115) formed on the substrate (102) and in communication with the data processing unit (110) to wirelessly transmit the processed data to a remote device (120),
wherein the pressure sensor (101c) includes a force sensitive resistor (FSR) or a strain gauge, and wherein the pressure sensor is operable to produce the electrical signal corresponding to an amount of pressure change caused by squeezing the body of the bottle container (435), and
wherein the sensor (101), the signal processing unit (105), the data processing unit 110, and the communications unit are implemented in a single device package.

2. The device as in claim 1, wherein the bottle container includes a droplet bottle (130) or a bottle (130) with an at least partially detachable lid (481).

3. The device as in claim 1, wherein the data processing unit (110) is configured to determine an amount of the medicine dispensed from the bottle container (130) based on the electrical signal.

4. The device as in claim 1, wherein the processed data indicates a time at which the determined dispensing event occurred, and wherein the data processing unit (110) is configured to determine if the time at which the determined dispensing event occurred is before or after a predetermined time.

5. The device as in claim 1, further comprising one or more of a temperature dependent resistive flow sensor (101a), an optical flow sensor (101b) or a position sensor (101d).

6. The device as in claim 5, wherein the medicine includes a fluid, and the substrate (102) is attached and conformed at or proximate to a nozzle of the bottle container (130), and wherein:
the temperature sensor (101a) includes a temperature dependent resistive flow sensor operable to produce the electrical signal by detecting a temperature fluctuation when the fluid passes out of the nozzle, and/or
the optical sensor (101b) includes a light emitting diode (LED) positioned at one end of the nozzle and a photodetector positioned at an opposing end of the nozzle to receive light emitted by the LED, wherein the optical sensor is operable to produce the electrical signal by detecting, at the photodetector, a change in a refraction index of the emitted light caused when the fluid passes out of the nozzle.

7. The device as in claim 6, wherein the data processing unit (110) is operable to determine if the detected amount of pressure is above or below a threshold value to determine the occurrence of the dispensing event.

8. The device as in claim 5, wherein the position sensor (101d) includes a gyroscope or an accelerometer, wherein the position sensor is operable, when the device is attached to the bottle container, to produce the electrical signal based on a change in orientation of the bottle container detected by the position sensor.

9. The device as in claim 1, further comprising:
an LED electrically coupled to the signal processing circuit (105) and operable, when the device is attached to the bottle container, to emit a light when the sensor detects the occurrence of the medicine dispensing from the bottle container.

10. The device as in claim 1, further comprising:
an alert unit including one or more of an LED, a speaker, or a vibrator in communication with the data processing unit (110) to generate, when the device is attached to the bottle container (130), an alert signal from the bottle container,
wherein the data processing unit is operable to determine compliance of a user of the bottle container for taking the medicine according to a schedule of use for the user, and to produce a command signal to actuate the alert unit to generate the alert signal when the dispensing event was early or late based on the schedule of use.

11. A method (170) for monitoring dispensing of medicine from a bottle container (130), comprising:
detecting (171) a signal by a sensor (101) on a conformal substrate attached and conformed to a body of a bottle container containing medicine including a fluid, wherein the conformal substrate includes a mechanically flexible and an electrically insulative material, and wherein the sensor includes a pressure sensor (101c);
processing (175), by a data processing unit (110) on the conformal substrate, the detected signal to produce data indicative of an occurrence of the medicine dispensed from the bottle container; and
wirelessly transmitting (173, 177), by a communications unit (115) formed on the conformal substrate and in communication with the data processing unit, the processed data to a remote device,
wherein the detecting includes transducing events corresponding to the medicine being dispensed from the bottle container into distinguishable electrical signals, and amplifying the electrical signals,
wherein the pressure sensor (101c) includes a force sensitive resistor (FSR) or a strain gauge, wherein the pressure sensor is operable to produce the electrical signals corresponding to an amount of pressure change caused by squeezing the body of the bottle container (435), and
wherein the sensor (101), the signal processing unit (105), the data processing unit 110, and the communications unit are implemented in a single device package.

12. The method as in claim 11, wherein the processing (175) includes determining an amount of the medicine dispensed from the bottle container (130) based on the detected signal, wherein the bottle container includes a droplet bottle (130) or a bottle (130) with an at least partially detachable lid (481).

13. The method as in claim 11, wherein the processing (175) includes associating a time value with a determined occurrence, wherein the processing includes determining if the time value associated with the determined occurrence is before or after a predetermined time.

14. The method as in claim 11, wherein the method further comprises:
producing (179) an alert corresponding to a message indicating a dispensing event is upcoming, a dispensing event has occurred, or a dispensing event has been missed.

15. The method as in claim 11, further comprising:
determining compliance of a user using the medicine according to a particular schedule of use for the user; and
generating a message indicating the dispensing event was early or late based on the predetermined time.

## Patentansprüche

1. Vorrichtung (100) zum Überwachen einer Patientencompliance einer medikamentösen Behandlung, die Folgendes umfasst:
ein Substrat (102), das zum Anbringen an einem Flaschenbehälter (130), der ein Medikament enthält, das ein Fluid beinhaltet, und zum Anpassen an diesen geeignet ist, wobei das Substrat ein mechanisch flexibles und ein elektrisch isolierendes Material beinhaltet;
eine Datenerhebungseinheit, die einen Sensor (101) und eine Signalverarbeitungsschaltung (105) beinhaltet, die auf dem Substrat ausgebildet sind, wobei der Sensor betriebsfähig ist, wenn die Vorrichtung an einem Körper des Flaschenbehälters angebracht ist, um ein Auftreten der Medikamentenabgabe aus dem Flaschenbehälter in ein elektrisches Signal zu wandeln, und die Signalverarbeitungsschaltung betriebsfähig ist, um das elektrische Signal zu verstärken, wobei der Sensor (101) einen Drucksensor (101c) beinhaltet;
eine Datenverarbeitungseinheit (110), die einen Prozessor (111) und einen Speicher (114) beinhaltet, die auf dem Substrat ausgebildet sind und mit der Datenerhebungseinheit in Kommunikation stehen, um das verstärkte elektrische Signal als Daten zu verarbeiten; und
eine Kommunikationseinheit (115), die auf dem Substrat (102) ausgebildet ist und in Kommunikation mit der Datenverarbeitungseinheit (110) steht, um die verarbeiteten Daten an eine entfernte Vorrichtung (120) drahtlos zu übertragen,
wobei der Drucksensor (101c) einen kraftempfindlichen Widerstand (*force sensitive resistor-* FSR) oder einen Dehnungsmessstreifen beinhaltet, und wobei der Drucksensor betriebsfähig ist, um das elektrische Signal zu erzeugen, das einer Menge einer Druckänderung entspricht, die durch Zusammendrücken des Körpers des Flaschenbehälters (435) verursacht wird, und
wobei der Sensor (101), die Signalverarbeitungseinheit (105), die Datenverarbeitungseinheit 110 und die Kommunikationseinheit in einem einzelnen Vorrichtungsgehäuse implementiert sind.

2. Vorrichtung nach Anspruch 1, wobei der Flaschenbehälter eine Tröpfchenflasche (130) oder eine Flasche (130) mit einem wenigstens teilweise abnehmbaren Deckel (481) beinhaltet.

3. Vorrichtung nach Anspruch 1, wobei die Datenverarbeitungseinheit (110) konfiguriert ist, um eine Menge des Medikaments, die aus dem Flaschenbehälter (130) abgegeben wird, basierend auf dem elektrischen Signal zu bestimmen.

4. Vorrichtung nach Anspruch 1, wobei die verarbeiteten Daten eine Zeit angeben, zu der das bestimmte Abgabeereignis aufgetreten ist, und wobei die Datenverarbeitungseinheit (110) konfiguriert ist, um zu bestimmen, ob die Zeit, zu der das bestimmte Abgabeereignis aufgetreten ist, vor oder nach einer zuvor bestimmten Zeit liegt.

5. Vorrichtung nach Anspruch 1, die ferner einen temperaturabhängigen Widerstandsdurchflusssensor (101a), einen optischen Durchflusssensor (101b) und/oder einen Positionssensor (101d) umfasst.

6. Vorrichtung nach Anspruch 5, wobei das Medikament ein Fluid beinhaltet und das Substrat (102) an oder nahe an einer Düse des Flaschenbehälters (130) angebracht ist und an diese angepasst ist und wobei:
der Temperatursensor (101a) einen temperaturabhängigen Widerstandsdurchflusssensor beinhaltet, der betriebsfähig ist, um das elektrische Signal durch Erfassen einer Temperaturschwankung zu erzeugen, wenn das Fluid aus der Düse austritt, und/oder
der optische Sensor (101b) eine Leuchtdiode (*light emitting diode* - LED), die an einem Ende der Düse positioniert ist, und einen Fotodetektor beinhaltet, der an einem gegenüberliegenden Ende der Düse positioniert ist, um Licht zu empfangen, das durch die LED emittiert wird, wobei der optische Sensor betriebsfähig ist, um das elektrische Signal durch Erfassen, an dem Fotodetektor, einer Änderung eines Brechungsindex des emittierten Lichts zu erzeugen, die verursacht wird, wenn das Fluid aus der Düse austritt.

7. Vorrichtung nach Anspruch 6, wobei die Datenverarbeitungseinheit (110) betriebsfähig ist, um zu bestimmen, ob die erfasste Druckmenge über oder unter einem Schwellenwert liegt, um das Auftreten des Abgabeereignisses zu bestimmen.

8. Vorrichtung nach Anspruch 5, wobei der Positionssensor (101d) ein Gyroskop oder einen Beschleunigungsmesser beinhaltet, wobei der Positionssensor betriebsfähig ist, wenn die Vorrichtung an dem Flaschenbehälter angebracht ist, um das elektrische Signal basierend auf einer Änderung einer Ausrichtung des Flaschenbehälters zu erzeugen, die durch den Positionssensor erfasst wird.

9. Vorrichtung nach Anspruch 1, die ferner Folgendes umfasst:
eine LED, die mit der Signalverarbeitungsschaltung (105) elektrisch gekoppelt ist und betriebsfähig ist, wenn die Vorrichtung an dem Flaschenbehälter angebracht ist, um ein Licht zu emittieren, wenn der Sensor das Auftreten der Medikamentenabgabe aus der Flasche erfasst.

10. Vorrichtung nach Anspruch 1, die ferner Folgendes umfasst:
eine Alarmeinheit, die eine oder mehrere LEDs, einen Lautsprecher oder einen Vibrator in Kommunikation mit der Datenverarbeitungseinheit (110) beinhaltet, um, wenn die Vorrichtung an dem Flaschenbehälter (130) angebracht ist, ein Alarmsignal von dem Flaschenbehälter zu generieren,
wobei die Datenverarbeitungseinheit betriebsfähig ist, um die Compliance eines Benutzers des Flaschenbehälters zum Einnehmen des Medikaments gemäß einem Verwendungsplan für den Benutzer zu bestimmen und ein Befehlssignal zu erzeugen, um die Alarmeinheit zu betätigen, um das Alarmsignal zu generieren, wenn das Ausgabeereignis basierend auf dem Verwendungsplan verfrüht oder verspätet erfolgt.

11. Verfahren (170) zum Überwachen der Abgabe des Medikaments aus einem Flaschenbehälter (130), das Folgendes umfasst:
Erfassen (171) eines Signals durch einen Sensor (101) auf einem konformen Substrat, das an einem Körper eines Flaschenbehälters, der ein Medikament enthält, das ein Fluid beinhaltet, angebracht und an diesen angepasst ist, wobei das konforme Substrat ein mechanisch flexibles und ein elektrisch isolierendes Material beinhaltet und wobei der Sensor einen Drucksensor (101c) beinhaltet;
Verarbeiten (175), durch eine Datenverarbeitungseinheit (110) auf dem konformen Substrat, des erfassten Signals, um Daten zu erzeugen, die ein Auftreten des Medikaments, das aus dem Flaschenbehälter abgegeben wird, anzugeben; und
drahtloses Übertragen (173, 177), durch eine Kommunikationseinheit (115), die auf dem konformen Substrat ausgebildet ist und in Kommunikation mit der Datenverarbeitungseinheit steht, der verarbeiteten Daten an eine entfernte Vorrichtung,
wobei das Erfassen das Wandeln von Ereignissen, die dem Abgeben des Medikaments aus dem Flaschenbehälter entsprechen, in unterscheidbare elektrische Signale und das Verstärken der elektrischen Signale beinhaltet,
wobei der Drucksensor (101c) einen kraftempfindlichen Widerstand (FSR) oder einen Dehnungsmessstreifen beinhaltet, wobei der Drucksensor betriebsfähig ist, um die elektrischen Signale zu erzeugen, die einer Menge der Druckänderung entsprechen, die durch Zusammendrücken des Körpers des Flaschenbehälters (435) verursacht wird, und
wobei der Sensor (101), die Signalverarbeitungseinheit (105), die Datenverarbeitungseinheit 110 und die Kommunikationseinheit in einem einzelnen Vorrichtungsgehäuse implementiert sind.

12. Verfahren nach Anspruch 11, wobei das Verarbeiten (175) das Bestimmen einer Menge des Medikaments, das aus dem Flaschenbehälter (130) abgegeben wird, basierend auf dem erfassten Signal beinhaltet, wobei der Flaschenbehälter eine Tröpfchenflasche (130) oder eine Flasche (130) mit einem wenigstens teilweise abnehmbaren Deckel (481) beinhaltet.

13. Verfahren nach Anspruch 11, wobei das Verarbeiten (175) ein Verknüpfen eines Zeitwerts mit einem bestimmten Auftreten beinhaltet, wobei das Verarbeiten das Bestimmen umfasst, ob der Zeitwert, der mit dem bestimmten Auftreten verknüpft ist, vor oder nach einer zuvor bestimmten Zeit liegt.

14. Verfahren nach Anspruch 11, wobei das Verfahren ferner Folgendes umfasst:
Erzeugen (179) eines Alarms, der einer Nachricht entspricht, die angibt, dass ein Ausgabeereignis bevorsteht, ein Ausgabeereignis aufgetreten ist oder ein Ausgabeereignis versäumt wurde.

15. Verfahren nach Anspruch 11, das ferner Folgendes umfasst:
Bestimmen der Compliance eines Benutzers, der das Medikament gemäß einem speziellen Verwendungsplan für den Benutzer verwendet; und
Generieren einer Nachricht, die angibt, dass das Ausgabeereignis basierend auf der zuvor bestimmten Zeit verfrüht oder verspätet war.

## Revendications

1. Dispositif (100) destiné à surveiller l'observance d'un médicament par le patient, comprenant :
un substrat (102) adapté pour se fixer et se conformer à un récipient de type bouteille (130) contenant un médicament comportant un fluide, dans lequel le substrat comporte un matériau mécaniquement flexible et un matériau électriquement isolant ;
une unité d'acquisition de données comportant un capteur (101) et un circuit de traitement de signal (105) formés sur le substrat, dans lequel le capteur peut fonctionner, lorsque le dispositif est fixé à un corps du récipient de type bouteille, pour transduire une occurrence de la distribution de médicament depuis le récipient de type bouteille en un signal électrique, et le circuit de traitement de signal peut fonctionner pour amplifier le signal électrique, dans lequel le capteur (101) comporte un capteur de pression (101c) ;
une unité de traitement de données (110) comportant un processeur (111) et une mémoire (114) formés sur le substrat et en communication avec l'unité d'acquisition de données pour traiter le signal électrique amplifié en tant que données ; et
une unité de communication (115) formée sur le substrat (102) et en communication avec l'unité de traitement de données (110) pour transmettre sans fil les données traitées à un dispositif distant (120),
dans lequel le capteur de pression (101c) comporte une résistance sensible à la force (FSR) ou une jauge de contrainte, et dans lequel le capteur de pression peut fonctionner pour produire le signal électrique correspondant à une quantité de changement de pression provoquée par la compression du corps du récipient de type bouteille (435), et
dans lequel le capteur (101), le l'unité de traitement de signal (105), l'unité de traitement de données (110) et l'unité de communication sont mises en œuvre dans un seul boîtier de dispositif.

2. Dispositif selon la revendication 1, dans lequel le récipient de type bouteille comporte une bouteille de gouttelettes (130) ou une bouteille (130) avec un couvercle au moins partiellement détachable (481).

3. Dispositif selon la revendication 1, dans lequel l'unité de traitement de données (110) est configurée pour déterminer une quantité du médicament distribuée à partir du récipient de type bouteille (130) sur la base du signal électrique.

4. Dispositif selon la revendication 1, dans lequel les données traitées indiquent un moment auquel l'événement de distribution déterminé s'est passé, et dans lequel l'unité de traitement de données (110) est configurée pour déterminer si le moment auquel l'événement de distribution déterminé s'est passé est avant ou après un moment prédéterminé.

5. Dispositif selon la revendication 1, comprenant en outre un capteur de débit résistif dépendant de la température (101a) et/ou un capteur de débit optique (101b) et/ou un capteur de position (101d).

6. Dispositif selon la revendication 5, dans lequel le médicament comporte un fluide, et le substrat (102) est attaché et conformé au niveau ou à proximité d'une buse du récipient de type bouteille (130), et dans lequel :
le capteur de température (101a) comporte un capteur de débit résistif dépendant de la température pouvant fonctionner pour produire le signal électrique en détectant une fluctuation de température lorsque le fluide sort de la buse, et/ou
le capteur optique (101b) comporte une diode électroluminescente (DEL) positionnée à une extrémité de la buse et un photodétecteur positionné à une extrémité opposée de la buse pour recevoir la lumière émise par la DEL, dans lequel le capteur optique peut fonctionner pour produire le signal électrique en détectant, au niveau du photodétecteur, une variation d'un indice de réfraction de la lumière émise provoquée lorsque le fluide sort de la buse.

7. Dispositif selon la revendication 6, dans lequel l'unité de traitement de données (110) peut fonctionner pour déterminer si la quantité de pression détectée est supérieure ou inférieure à une valeur de seuil pour déterminer l'occurrence de l'événement de distribution.

8. Dispositif selon la revendication 5, dans lequel le capteur de position (101d) comporte un gyroscope ou un accéléromètre, dans lequel le capteur de position peut fonctionner, lorsque le dispositif est fixé au récipient de type bouteille, pour produire le signal électrique sur la base d'un changement d'orientation du récipient de type bouteille détecté par le capteur de position.

9. Dispositif selon la revendication 1, comprenant en outre :
une DEL couplée électriquement au circuit de traitement de signal (105) et pouvant fonctionner, lorsque le dispositif est fixé au récipient de type bouteille, pour émettre une lumière lorsque le capteur détecte l'occurrence de la distribution de médicament à partir du récipient de type bouteille.

10. Dispositif selon la revendication 1, comprenant en outre :
une unité d'alerte comportant une DEL et/ou un haut-parleur et/ou un vibreur en communication avec l'unité de traitement de données (110) pour générer, lorsque le dispositif est fixé au récipient de type bouteille (130), un signal d'alerte provenant du récipient de type bouteille,
dans lequel l'unité de traitement de données peut fonctionner pour déterminer l'observance d'un utilisateur du récipient de type bouteille pour prendre le médicament selon un calendrier d'utilisation pour l'utilisateur, et pour produire un signal de commande afin d'actionner l'unité d'alerte pour générer le signal d'alerte lorsque l'événement de distribution était précoce ou tardif sur la base du calendrier d'utilisation.

11. Procédé (170) destiné à surveiller la distribution d'un médicament à partir d'un récipient de type bouteille (130), comprenant :
la détection (171) d'un signal par un capteur (101) sur un substrat conforme fixé et conformé à un corps d'un récipient de type bouteille contenant un médicament comportant un fluide, dans lequel le substrat conforme comporte un matériau mécaniquement flexible et un matériau électriquement isolant, et dans lequel le capteur comporte un capteur de pression (101c) ;
le traitement (175), par une unité de traitement de données (110) sur le substrat conforme, du signal détecté pour produire des données indiquant une occurrence du médicament distribué à partir du récipient de type bouteille ; et
la transmission sans fil (173, 177), par une unité de communication (115) formée sur le substrat conforme et en communication avec l'unité de traitement de données, des données traitées à un dispositif distant,
dans lequel la détection comporte la transduction d'événements correspondant au médicament distribué à partir du récipient de type bouteille en signaux électriques distinctifs, et l'amplification des signaux électriques, dans lequel le capteur de pression (101c) comporte une résistance sensible à la force (FSR) ou une jauge de contrainte,
dans lequel le capteur de pression peut fonctionner pour produire les signaux électriques correspondant à une quantité de changement de pression provoquée par la compression du corps du récipient de type bouteille (435), et
dans lequel le capteur (101), l'unité de traitement de signal (105), l'unité de traitement de données 110 et l'unité de communication sont mises en œuvre dans un seul boîtier de dispositif.

12. Procédé selon la revendication 11, dans lequel le traitement (175) comporte la détermination d'une quantité du médicament distribuée à partir du récipient de type bouteille (130) sur la base du signal détecté, dans lequel le récipient de type bouteille comporte une bouteille à gouttelettes (130) ou une bouteille (130) avec un couvercle au moins partiellement détachable (481).

13. Procédé selon la revendication 11, dans lequel le traitement (175) comporte l'association d'une valeur de temps à une occurrence déterminée, dans lequel le traitement comporte la détermination de savoir si la valeur de temps associée à l'occurrence déterminée est avant ou après un moment prédéterminé.

14. Procédé selon la revendication 11, dans lequel le procédé comprend en outre :
la production (179) d'une alerte correspondant à un message indiquant qu'un événement de distribution est imminent, qu'un événement de distribution s'est produit ou qu'un événement de distribution a été manqué.

15. Procédé selon la revendication 11, comprenant en outre :
la détermination de la conformité d'un utilisateur utilisant le médicament selon un calendrier d'utilisation particulier pour l'utilisateur ; et
la génération d'un message indiquant que l'événement de distribution était en avance ou en retard sur la base du moment prédéterminé.
